# EUROPEAN PATENT APPLICATION

(11) **EP 4 811 383 A1**
(43) Date of publication of application: **23.09.2026**
(21) Application number: 25164918.2
(22) Date of filing: 20.03.2025
(51) Int. Cl.: G16H 30/40, A61B 6/51, G16H 30/20, G16H 50/20, G16H 20/00, G16H 50/30

(54) **METHOD FOR COMPARING DIGITAL 3D DENTAL MODELS IN A MOBILE APPLICATION**

(71) Applicant: 3Shape A/S, 1060 Copenhagen K (DK)
(72) Inventor: BREHMER, Kasper, 1060 Copenhagen K (DK); MANSFELDT, Ole Kjær, 1060 Copenhagen K (DK); NIELSEN, Malene Leonora, 1060 Copenhagen K (DK)
(74) Representative: Zacco Denmark A/S

(57) **Abstract**

A computer-implemented method for comparing digital 3D dental models of a patient's oral situation in a user interface (600) of a mobile application is disclosed, the method comprising receiving, in the user interface (600) of the mobile application, a selection of a dental condition of the at least two dental conditions, displaying buttons (602, 603, 604) representative of digital 3D dental models available for comparison, wherein the buttons comprise a first button (602) associated with a first digital 3D dental model (601) representative of a patient's oral situation at a first point in time and a second button (603) associated with a second digital 3D dental model representative of the patient's oral situation at a second point in time. The method further comprises receiving, in the user interface (600), a selection of the first button (602), and in response, displaying the first digital 3D dental model (601) in the user interface (600), such that the displayed first digital 3D dental model (601) visualizes the selected dental condition corresponding to the first point in time. Further, the method comprises launching a compare mode of the mobile application by detecting a drag command performed by a user of the mobile application, the drag command comprising a drag motion, in the user interface (600), from a compare button (606) to the second button (603), and in response to the drag command, displaying a transition from the first digital 3D dental model (601) to the second digital 3D dental model, wherein the second digital 3D dental model visualizes the selected dental condition corresponding to the second point in time.

## Description

### Technical field

The disclosure relates to a computer-implemented method for comparing digital 3D dental models of a patient's oral situation in a user interface of the mobile application and the corresponding user interface. Furthermore, the disclosure relates to computer-implemented method for comparing, in a user interface of a mobile application, the digital 3D dental model of the patient's oral situation to the user's oral situation and the corresponding user interface. The disclosure further relates to a computer-implemented method and system for determining an individual dental profile of a patient based on diagnostic intraoral scan data of the patient's oral situation. In particular, the individual dental profile is generated by a mobile application which provides curated patient-specific metadata based on the individual dental profile.

### Background

Development of intraoral scanning techniques has been instrumental in the transition to modern digital dentistry. Use of intraoral 3D scanners allows dental practitioners to accurately and quickly capture scan data representative of a patient's oral situation, which may then be visualized on a display device as a digital three-dimensional (3D) dental model. Obtained digital 3D dental model may thus serve as a digital impression of teeth, offering numerous advantages over a classical physical impression of teeth.

The obtained scan data and/or the corresponding digital 3D model may be analysed, by means of various algorithms, for presence of commonly-occurring dental conditions such as caries, tooth wear, dental plaque, gingival recession and similar. These powerful diagnostic tools nor the results obtained by their use are currently not available to patients. Thus, the patients suffering from these dental conditions often don't have in-depth insight into their diagnostic data and may only be exposed to limited information conveyed by their dentists.

The challenge therefore exists to create solutions for sharing patient-specific diagnostic information, educate patients to understand their dental health, provide care guidance, boost treatment acceptance and overall, to increase patient engagement with respect to their dental health.

The present disclosure investigates how to develop methods, systems, a mobile application and graphical user interfaces (GUIs) to provide patients with a diagnostic overview of various dental conditions and to provide tracking of dental conditions over time. Furthermore, the disclosure addresses how to develop and provide a personalised oral care guidance for the patient where self-reporting on dental issues is facilitated. Moreover, the disclosure deals with improving the overall interaction between a dentist and a patient, such as appointment setting, offering and selecting appropriate dental treatments etc.

### Summary

In an embodiment a computer-implemented method is described, the method comprising:
- receiving, by a mobile application, a diagnostic intraoral scan data of a patient's oral situation, wherein the diagnostic intraoral scan data comprises a digital 3D dental model of the patient's oral situation, a quantitative data for at least two dental conditions associated with the patient's oral situation, a qualitative data for the at least two dental conditions, and a mesh overlay for each of the at least two dental conditions, wherein the mesh overlay is configured, when applied to the digital 3D dental model, to visualize the quantitative data and the qualitative data on the digital 3D dental model,
- generating, by the mobile application, an individual dental profile for the patient based on the quantitative data and the qualitative data,
- obtaining, by the mobile application, metadata associated with the at least two dental conditions, wherein the metadata is curated based on the individual dental profile,
- displaying, in a user interface of the mobile application, the digital 3D dental model with the applied mesh overlay, and further displaying the metadata.

The mobile application may be a specially designed mobile application for generating and presenting dental health status to a user of the mobile application.

Expression "3D" throughout the present disclosure refers to a term "three-dimensional". Term "digital 3D dental model" refers to a digital, three-dimensional, computer-generated representation of the patient's oral situation.

Such digital 3D dental model may accurately correspond to the patient's actual dental situation. That means that dental objects like teeth, teeth surfaces, restorations and/or gingiva of the digital 3D dental model may correspond to those of the patient's dental situation.

The digital 3D dental model may be constructed by a processor, based on scan data (also referred to as intraoral scan data) collected in an intraoral scanning process in which an intraoral 3D scanner may be used to scan the patient's oral situation comprising teeth and gingiva. The intraoral 3D scanner throughout the disclosure is also referred to as the intraoral scanner. The digital 3D dental model can be stored in a memory of a computer system, for example in a Standard Triangle Language (STL) format or any other format suitable for viewing and/or printing 3D objects.

The scan data may comprise different type of data acquired by the intraoral scanner, for example one or more of: 3D geometric data, color data, infrared (IR) data, fluorescence data.

The digital 3D dental model can be received or accessed by the processor. The digital 3D dental model may usually be displayed on a display screen in form of a 3D mesh, representing surfaces of teeth and gingival tissue of the oral situation. The 3D mesh may be comprised of individual facets, for example triangular facets while each facet may comprise, for example, three mutually connected vertices. Alternatively, the digital 3D dental model may be displayed as a point cloud comprising points, a graph comprising nodes and edges, a volumetric representation comprising voxels, or any other suitable 3D representation form.

The diagnostic intraoral scan data of the patient's oral situation may be obtained by processing the scan data to identify presence of dental conditions in the patient's oral situation. For example, the scan data may be processed by a diagnostic algorithm capable of detecting dental conditions in the scan data. The diagnostic algorithm may be, for example, a trained machine learning (ML) algorithm comprising one or more neural networks trained for detecting dental conditions. Alternatively, the diagnostic algorithm may be a classical algorithm based on predefined rules (non-ML algorithm).

The at least two dental conditions may be at least two of: caries, tooth wear, dental plaque, gingival recession, gingivitis, periodontitis, tooth cracks. Instead of working with the at least two dental conditions, the mobile application may provide, in some embodiments, only information on a single dental condition.

The diagnostic intraoral scan data may comprise the quantitative data for the at least two dental conditions associated with the patient's oral situation. The quantitative data may comprise a number of teeth affected for each of the at least two dental conditions in the patient's oral situation. The number of teeth affected may be a percentage of total tooth surfaces if a dental condition of the at least two dental conditions is dental plaque. The qualitative data on the other hand may comprise severity values for each of the at least two dental conditions in the patient's oral situation. The severity values may be defined for individual teeth and/or gingiva of the digital 3D dental model.

The quantitative data for a dental condition may therefore indicate a number of teeth, or a percentage of total tooth surfaces affected by that particular dental condition. For example, the quantitative data for caries may indicate that 8 teeth are affected by caries, although not providing information on how severe those caries cases may be. In another example, the quantitative data for dental plaque may indicate that 35% of total tooth surfaces are affected by dental plaque. Tooth surfaces may be an occlusal surface, a lingual surface, a buccal surface, a mesial surface and/or a distal surface.

The diagnostic intraoral scan data may comprise the qualitative data for the at least two dental conditions. The qualitative data for a dental condition may indicate a severity level of that dental condition. For example, out of 8 teeth affected by caries, 2 teeth may have initial caries, 5 teeth may have moderate caries and 1 tooth may have severe caries. Similar severity levels ("initial", "moderate", "severe") may be applicable to other dental conditions. In the case of dental plaque, the severity levels may be "thin plaque" and "thick plaque". Generally, the severity levels may correspond to clinically-adopted severity levels of various dental conditions.

The diagnostic intraoral scan data may additionally comprise the mesh overlay for each of the at least two dental conditions, wherein the mesh overlay is configured, when applied to the digital 3D dental model, to visualize the quantitative data and the qualitative data on the digital 3D dental model. The mesh overlay may thus be a 3D color overlay that may be applied on the surface of the digital 3D dental model. Colors of the mesh overlay may indicate parts of the digital 3D dental model affected by a dental condition as well as the severity level of that dental condition. For example, colors such as yellow, orange and red may be used to highlight different severity levels of the dental conditions. The mesh overlay may, instead of colors, use patterns or various textures to signify different severity levels.

The method according to the disclosure may comprise generating, by the mobile application, the individual dental profile for the patient based on the quantitative data and the qualitative data. The individual dental profile generated in this manner is reflective of the patient's dental health at a certain time. Generation of the individual dental profile enables subsequent creation of a personalized care plan for the dental health of the patient.

Generating the individual dental profile may comprise determining a representative severity value for each of the at least two dental conditions. Therefore, the representative severity value may be determined for at least two of caries, tooth wear, dental plaque, gingival recession, gingivitis, periodontitis, tooth cracks. The individual dental profile in this manner clearly defines the state of the patient's dental health with respect to the at least two dental conditions. In an example, the representative severity value for each of the at least two dental conditions may be computed as a weighted average of the quantitative data and the qualitative data for the each of the at least two dental conditions. The individual dental profile may then comprise the representative severity values for each of the at least two dental conditions or may be determined as a function of the representative severity values.

The method may further comprise displaying, in the user interface of the mobile application, the individual dental profile. The individual dental profile may be displayed in form of a single dental score which may be a numerical value. The dental score may be determined as a function of the representative severity values of the at least two dental conditions, such as a weighted average, taking a maximum of the representative severity values, taking the majority of the representative severity values etc. Displaying of the individual dental profile as the single dental score may be advantageous as it can convey to the user the holistic status of the dental health, in form of a single value.

In an embodiment, the dental score may be determined based on data collected from other users of the mobile application. The data collected from the other users of the mobile application may comprise individual dental profiles of the other users. In this way the dental score may indicate to the patient the dental health relative to other patients, for example in the same geographic area and/or relative to other patients of the same or similar age, for example within 2 or 3 years of age difference.

The term "user interface" throughout the disclosure may relate to a graphical user interface (GUI) of the mobile application.

The method may further comprise obtaining ranked dental conditions by ranking the at least two dental conditions based on the quantitative data and/or the qualitative data. Alternatively, the ranked dental conditions may be obtained by ranking the at least two dental conditions based on the representative severity values of the at least two dental conditions. Obtaining the ranked dental conditions is advantageous because this information can subsequently be displayed to the user of the mobile application, for example as an ordered list. This is particularly beneficial considering a limited area for displaying information on the display screen of the mobile phone hosting the mobile application, where ranking of the dental conditions may be the efficient way to present relative severity relationship of the at least two dental conditions. Thereby, a complete overview of dental conditions may be provided, which allows the user to easily identify and focus on the most serious dental condition first. The most serious dental condition may be the one with the highest representative severity value and may be ranked first in the list.

In an embodiment, the ranked dental conditions may be obtained by ranking the at least two dental conditions based on a change in quantitative data and/or the qualitative data of each of the at least two dental conditions over time. Thus, the dental condition which deteriorated more may be ranked higher compared to the dental conditions with lesser change.

The ranked dental conditions may be displayed in the user interface of the mobile application. Generally, the mobile application may be hosted on a mobile device, such as a mobile phone or a tablet. A highest ranked dental condition of the ranked dental conditions may be the one with the highest representative severity value. In addition to the ranked dental conditions, the digital 3D dental model may be displayed.

For example, the displayed digital 3D dental model may comprise the applied mesh overlay highlighting the highest ranked dental condition of the ranked dental conditions. Therefore, the mesh overlay may correspond to the qualitative data and/or quantitative data associated with the highest ranked dental condition. In this way, the user is presented with the visual representation of the most severe dental condition. The mesh overlay may update based on user selection of a dental condition other than the highest ranked dental condition, to visualize the selected dental condition.

The displayed digital 3D dental model with the applied mesh overlay may indicate parts of the digital 3D dental model affected by the highest ranked dental condition or otherwise selected dental condition. This may be achieved through applying various colors, patterns and/or textures in the mesh overlay which represent location and/or severity levels of the highest ranked dental condition.

In an embodiment, the displayed metadata may correspond to the highest ranked dental condition of the ranked dental conditions. In an example where the highest ranked dental condition is dental plaque, the metadata may comprise information on dental plaque present in the patient's oral situation. Generally, the metadata may comprise descriptive information for the at least two dental conditions and/or a personalized care plan for each of the at least two dental conditions. The personalized care plan may include, depending on the individual dental profile, statements such as "Pay attention!", "Keep an eye!", "Nothing to worry!" or similar. Additionally, the personalized care plan may include actionable recommendations such as, in an example of dental plaque, to change tooth brushing technique, increase a frequency of flossing, visit a dentist etc. These are only non-limiting examples. The metadata, and with it the personalized care plan may be specific to a dental condition and furthermore specific to the individual dental profile.

The method may further comprise receiving a user selection of a dental condition other than the highest ranked dental condition and updating the applied mesh overlay such that the digital 3D dental model visualizes the selected dental condition. In addition to updating the mesh overlay, the displayed metadata may be updated to reflect the selected dental condition. In this way the user can receive the metadata with the personalized care plan specific to the selected dental condition.

The method may further comprise updating the metadata such that the displayed metadata corresponds to the selected dental condition. The metadata may be obtained from an official clinical source such as World Health Organization (WHO), American Dental Association (ADA), National Health Service (NHS) or similar. The official clinical source may therefore be a local, regional, national or global health organization addressing human dental health.

To maximize the positive impacts of the mobile application to the user it is particularly important to generate metadata tailored for the specific individual dental profile. Therefore, the method may further comprise curating the metadata obtained from the official clinical source by identifying correspondences of the metadata with the individual dental profile. Term curating may relate to purposely selecting and organizing the metadata for presentation to the user. The correspondences in this context may relate to correspondences between a representative severity value of a dental condition and metadata associated with such representative severity value of that dental condition. As an effect, only that information from the official clinical source which is relevant to the actual dental health of the patient is obtained. For example, if the representative severity value for the dental plaque is "moderate", then only information relevant to this particular condition is sought after. Alternatively or additionally, the metadata may be curated based on the qualitative data and/or quantitative data of the diagnostic intraoral scan data.

The method may comprise receiving, in the user interface, a user selection of a first dental condition of the at least two dental conditions and updating the user interface to display the metadata associated with the first dental condition. Furthermore, the method may comprise receiving a user selection of a second dental condition of the at least two dental conditions, updating the displayed digital 3D dental model with a corresponding mesh overlay such that the digital 3D dental model visualizes the second dental condition and updating the user interface to display the metadata associated with the second dental condition. The user selection may be registered as a swipe motion on the display screen of the mobile phone hosting the mobile application. In this way the dental conditions with associated metadata can be presented as cards in the user interface that may be navigated through with a swipe motion by the user.

In an embodiment, the method may further comprise annotating the diagnostic intraoral scan data with a demographic data of the patient. Demographic data may comprise age, gender, ethnicity, education and/or marital status. This data may be incorporated into the individual dental profile. The method may therefore further comprise updating the individual dental profile with the demographic data of the patient. This allows characterizing the patient in a group or a sub-group of individuals based on the demographic data, for example classifying the patient in a specific age group.

The method may further comprise benchmarking the updated individual dental profile based on at least part of the demographic data. For example, the representative severity levels of the at least two dental conditions of the patient may be benchmarked against those of the same or similar age group, same country, gender, nationality etc.

Benchmarking of the updated individual dental profile may be implemented, in an example, in the following way. The diagnostic intraoral scan data of the patient may be stored in a diagnostic database together with diagnostic intraoral scan data of a plurality of other patients. This data may be anonymised and may be unusable for benchmarking unless the patient consents to providing their corresponding demographic data. Following a user input, the mobile application may provide the demographic data to the diagnostic database. The demographic data may be associated with the diagnostic intraoral scan data of the patient. Subsequent to this association, sufficient information is available for benchmarking of the patient's individual dental profile against individual dental profiles of the plurality of other patients.

The method according to the disclosure may further comprise receiving, by the mobile application, an external signal, and based on the received external signal deleting the quantitative and/or qualitative data for at least one dental condition of the at least two dental conditions. The external signal may come from a dentist for example when assessed that sending certain quantitative and/or qualitative data is unnecessary for the user to see. For example, it may be unnecessary to send information on severity of tooth wear if the same severity is constant over a defined time period and tooth wear has not worsened. The effect of this feature is that an external party, such as the dentist, may impact the metadata content presented to the user of the mobile application. Instead of deleting the quantitative and/or qualitative data, that data may otherwise be removed from displaying to the user. The effect of this feature is that the dentist may affect what information is finally displayed to the user.

In an example, the method may further comprise updating the user interface with a request for user's input on dental habits, receiving user's input on dental habits, and generating a recommended dental routine based on the user's input on dental habits and/or the individual dental profile. The request for user's input on dental habits may be in form of a questionnaire. The dental habits may relate to frequency of tooth brushing, use of dental floss, type of toothbrush used, use of mouthwash products, frequency of dentist visits etc. In one example the recommended dental routine may be generated solely based on the user's input on dental habits or solely based on the individual dental profile. In an advantageous example, the recommended dental routine may be generated based on both the user's input on dental habits and the individual dental profile. By considering the user's input on dental habits, the root-cause for a dental condition of the at least two dental conditions may be uncovered. The recommended dental routine may be displayed, in the user interface, for each of the at least two dental conditions. The recommended dental routine may also be referred to as the personalized care plan for the user. This plan may comprise actionable recommendations for treatment or containment of the dental condition. Generating of the recommended dental routine may comprise accessing the official clinical source. Actionable recommendations may be generated by accessing the official clinical source and retrieving information relevant to the dental condition.

In an embodiment, the method may further comprise updating the individual dental profile based on the user's input on dental habits. In this way the user may directly contribute to development of their individual dental profile.

In an example the recommended dental routine may be displayed with a button, which when engaged, initiates a dialogue with the dentist, for example opens a chat communication mode. In this way, the user may obtain a direct communication channel with the dentist.

The method may additionally or alternatively comprise receiving a user input concerning dental symptoms and updating the individual dental profile based on the received user input concerning dental symptoms. Dental symptoms may include tooth ache, sore jaw, blood etc. Based on receiving the user input concerning dental systems, the metadata and/or the recommended dental routine may be updated.

Disclosed is further a data processing apparatus comprising means for carrying out a method comprising:
- receiving, by the mobile application, the diagnostic intraoral scan data of the patient's oral situation, wherein the diagnostic intraoral scan data comprises the digital 3D dental model of the patient's oral situation, the quantitative data for the at least two dental conditions associated with the patient's oral situation, the qualitative data for the at least two dental conditions, and the mesh overlay for each of the at least two dental conditions, wherein the mesh overlay is configured, when applied to the digital 3D dental model, to visualize the quantitative data and the qualitative data on the digital 3D dental model,
- generating, by the mobile application, the individual dental profile for the patient based on the quantitative data and the qualitative data,
- obtaining, by the mobile application, metadata associated with the at least two dental conditions, wherein the metadata is curated based on the individual dental profile,
- displaying, in the user interface of the mobile application, the digital 3D dental model with the applied mesh overlay, and further displaying the metadata.

A computer program product is further disclosed, comprising instructions which, when the program is executed by a processor, cause the processor to carry out a method comprising:
- receiving, by the mobile application, the diagnostic intraoral scan data of the patient's oral situation, wherein the diagnostic intraoral scan data comprises the digital 3D dental model of the patient's oral situation, the quantitative data for the at least two dental conditions associated with the patient's oral situation, the qualitative data for the at least two dental conditions, and the mesh overlay for each of the at least two dental conditions, wherein the mesh overlay is configured, when applied to the digital 3D dental model, to visualize the quantitative data and the qualitative data on the digital 3D dental model,
- generating, by the mobile application, the individual dental profile for the patient based on the quantitative data and the qualitative data,
- obtaining, by the mobile application, metadata associated with the at least two dental conditions, wherein the metadata is curated based on the individual dental profile,
- displaying, in the user interface of the mobile application, the digital 3D dental model with the applied mesh overlay, and further displaying the metadata.

A computer-readable medium is further disclosed comprising instructions which, when executed by a processor, cause the processor to carry out the method according to the disclosure. The computer-readable medium may be a non-transitory computer-readable medium. The method may comprise:
- receiving, by the mobile application, the diagnostic intraoral scan data of the patient's oral situation, wherein the diagnostic intraoral scan data comprises the digital 3D dental model of the patient's oral situation, the quantitative data for the at least two dental conditions associated with the patient's oral situation, the qualitative data for the at least two dental conditions, and the mesh overlay for each of the at least two dental conditions, wherein the mesh overlay is configured, when applied to the digital 3D dental model, to visualize the quantitative data and the qualitative data on the digital 3D dental model,
- generating, by the mobile application, the individual dental profile for the patient based on the quantitative data and the qualitative data,
- obtaining, by the mobile application, metadata associated with the at least two dental conditions, wherein the metadata is curated based on the individual dental profile,
- displaying, in the user interface of the mobile application, the digital 3D dental model with the applied mesh overlay, and further displaying the metadata.

In an embodiment, a system is disclosed, the system comprising:
- a mobile application,
- a content management system,
- a diagnostic module configured to generate a diagnostic intraoral scan data of a patient, wherein the mobile application is configured to:
- receive, from the diagnostic module, the diagnostic intraoral scan data of the patient comprising a digital 3D dental model,
- generate an individual dental profile for the patient based on the intraoral scan data,
- obtain, from the content management system, metadata based the individual dental profile,
- display, in a user interface of the mobile application, the digital 3D dental model and the metadata.

The content management system may represent the system entity responsible for generating specific information content packages for the mobile application as well as for delivery of the content packages and updates to the content packages of the mobile application.

The content management system may be configured to provide metadata to the mobile application with a time delay. The time delay may be selected so to correspond to a treatment regime that may be comprised in the metadata. The time delay may alternatively or additionally be determined based on the individual dental profile.

The content management system may be configured to instruct the mobile application to request the user's input on dental habits and/or dental symptoms. The content management system may further be configured to curate the metadata before providing the metadata to the mobile application. Curating the metadata may be performed by accessing an official clinical source.

The diagnostic module may be tasked with processing intraoral scan data of the patient and generating the diagnostic intraoral scan data indicating presence and/or severity of the at least two dental conditions. The diagnostic intraoral scan data may comprise the digital 3D dental model of the patient's oral situation, the quantitative data for the at least two dental conditions associated with the patient's oral situation, the qualitative data for the at least two dental conditions, and the mesh overlay for each of the at least two dental conditions, wherein the mesh overlay is configured, when applied to the digital 3D dental model, to visualize the quantitative data and the qualitative data on the digital 3D dental model.

The diagnostic module may be configured to compress the digital 3D dental model before sending the digital 3D dental model to the mobile application. In this way the mobile application may process 3D datafiles of lower size and thereby increase the processing speed. The mobile application may alternatively be configured to compress the digital 3D dental model before storing the digital 3D dental model to a memory of the mobile device hosting the mobile application.

In an embodiment, a computer-implemented method for comparing digital 3D dental models of a patient's oral situation in a user interface of a mobile application is disclosed, the method comprising:
- receiving, in the user interface of the mobile application, a selection of a dental condition of the at least two dental conditions,
- displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time,
- receiving, in the user interface, a selection of the first button, and in response, displaying the first digital 3D dental model in the user interface, such that the displayed first digital 3D dental model visualizes the selected dental condition corresponding to the first point in time,
- launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.

The presented method for comparing digital 3D dental models of the patient's oral situation in the user interface of the mobile application is advantageous as it solves a problem of arranging and presenting comparison of digital 3D dental models on the mobile phone. Considering a limited touchscreen area of the mobile phone hosting the mobile application, the presented solution effectively guides the user through the process of selecting digital 3D dental models for comparison and presenting such comparison.

The mobile application and the user interface referred to in the method for comparing digital 3D dental models of the patient's oral situation may be the same as described throughout the disclosure and with respect to other methods mentioned herein.

This described embodiment of comparing digital 3D dental models of the patient's oral situation describes use of the drag command on the touchscreen of the mobile device hosting the mobile application for the purpose of comparing digital 3D dental models. Comparison of the user's various digital 3D dental models from different times is relevant for the user to visualize changes in dental conditions over time. This disclosed method effectively utilizes limited surface of the touchscreen economically and provides for time-efficient way of comparing the digital 3D dental models.

The transition from the first digital 3D dental model to the second digital 3D dental model may comprise morphing the first digital 3D dental model into the second digital 3D dental model. Morphing may be understood as morphing of 3D geometry from one shape into another, for example a geometric transformation describing the transition of vertices of the first digital 3D dental model to corresponding vertices of the second digital 3D dental model. Alternatively, the transition from the first digital 3D dental model to the second digital 3D model may comprise adjusting a transparency level of the first digital 3D dental model to lower visibility of the first digital 3D dental model and adjusting a transparency level of the second digital 3D dental model to increase visibility of the second digital 3D dental model. This transparency adjustment may be done simultaneously so that the user may perceive the seamless change of 3D geometries.

The transition from the first digital 3D dental model to the second digital 3D dental model may highlight a difference between the first digital 3D dental model and the second digital 3D dental model, wherein the difference corresponds to a change in the selected dental condition.

The compare mode may be active during the user's application of the drag command. Once the user disengages from the touchscreen, the drag command stops and the compare mode may be exited. Upon exiting of the compare mode, the display screen may show transitioning back to the first digital 3D dental model. The method may thus comprise detecting cessation of the drag command, and in response displaying a transition from the second digital 3D dental model to the first digital 3D dental model.

During the compare mode, the first and/or the second digital 3D dental model may be displayed with jaws closed. Based on receiving user input, for example through a dedicated button in the user interface, the first and/or the second digital 3D dental model may be displayed from an occlusal view (i.e. the jaws of the digital 3D dental models may be open).

During the compare mode and during displaying of the second digital 3D dental model, the second digital 3D dental may be rotated such that a difference between the first digital 3D dental model and the second digital 3D dental model is in a field of view of the user. As there may be a plurality of differences between the two digital 3D models, the one difference designated for displaying may be the one with the highest severity value and/or surface area.

The first digital 3D dental model and the second digital 3D dental model may be stored on a memory of the mobile device hosting the mobile application or alternatively on a remote memory outside the mobile device in which case the mobile application is configured to access the digital 3D dental models.

Furthermore, the user interface of the mobile application for tracking dental health of the patient is disclosed, the user interface comprising:
- buttons representative of digital 3D dental models available for comparison, wherein the buttons representative of digital 3D dental models comprise a first button associated with a first digital 3D dental model representative of a patient's oral situation at a first point in time and a second button associated with a second digital 3D dental model representative of the patient's oral situation at a second point in time, wherein the user interface is configured for displaying the first digital 3D dental model based on a user selection of the first button,
- a compare button, wherein the user interface is configured to launch a compare mode of the mobile application by detecting a drag command performed by the user of the mobile application, the drag command comprising a drag motion, in the user interface, from the compare button to the second button, and in response to the drag command, display a transition from the first digital 3D dental model to the second digital 3D dental model.

The user interface may further comprise a button representative of a dental condition, wherein the user interface is configured to visualize the dental condition on the first digital 3D dental model or the second digital 3D dental model, based on the user selection of the button representative of the dental condition. This functionality offers the user a choice of selecting which dental condition they would like to visualize on the displayed digital 3D dental model.

The dental condition may be visualized as a surface color overlay or alternatively, as a pattern or texture overlay.

The user interface may be configured to display the transition from the first digital 3D dental model to the second digital 3D dental model, which may be a geometry morphing of the first digital 3D dental model into the second digital 3D dental model.

A data processing apparatus is further disclosed comprising means for carrying out a method comprising:
- receiving, in the user interface of the mobile application, a selection of a dental condition of the at least two dental conditions;
- displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time;
- receiving, in the user interface, a selection of the first button, and in response, displaying the first digital 3D dental model in the user interface, such that the displayed first digital 3D dental model visualizes the selected dental condition corresponding to the first point in time;
- launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.

A computer program product is further disclosed, comprising instructions which, when the program is executed by a processor, cause the processor to carry out the method comprising:
- receiving, in the user interface of the mobile application, a selection of a dental condition of the at least two dental conditions;
- displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time;
- receiving, in the user interface, a selection of the first button, and in response, displaying the first digital 3D dental model in the user interface, such that the displayed first digital 3D dental model visualizes the selected dental condition corresponding to the first point in time;
- launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.

A computer-readable medium is further disclosed, comprising instructions which, when executed by a processor, cause the processor to carry out the method comprising:
- receiving, in the user interface of the mobile application, a selection of a dental condition of the at least two dental conditions;
- displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time;
- receiving, in the user interface, a selection of the first button, and in response, displaying the first digital 3D dental model in the user interface, such that the displayed first digital 3D dental model visualizes the selected dental condition corresponding to the first point in time;
- launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.

After the user receives and inspects the status of a dental condition in the mobile application, they may want to simultaneously observe their actual oral situation in order to try to detect the dental condition visualized in the mobile application. The disclosure, in an embodiment, presents a computer-implemented method for comparing, in a user interface of a mobile application, a digital 3D dental model of a patient's oral situation to a user's oral situation, the method comprising:
- receiving, in the user interface, a selection of a dental condition of the at least two dental conditions, and displaying buttons representative of digital 3D dental models available for comparison, wherein the buttons comprise a first button associated with a first digital 3D dental model representative of a patient's oral situation at a first point in time and a second button associated with a second digital 3D dental model representative of a patient's oral situation at a second point in time,
- receiving, in the user interface, a selection of the first button, and in response, displaying the first digital 3D dental model in a first area of the user interface, such that the displayed first digital 3D dental model visualizes the selected dental condition corresponding to the first point in time,
- arranging, in the user interface, a camera button configured, when engaged, to activate a front camera of a mobile device hosting the mobile application,
- detecting engaging of the camera button by a user, and in response, activating the front camera,
- displaying, in a second area of the user interface, a camera stream from the front camera such that the user, when facing the front camera, may compare the user's oral situation to the first digital 3D dental model.

The user may in this way see both their actual oral situation and the displayed digital 3D model visualizing the relevant dental condition. In this way, the user is presented with a unique solution to simultaneously observe the detected dental condition and their actual oral situation.

The mobile application and the user interface referred to in the method for comparing digital 3D dental model of the patient's oral situation to the user's actual oral situation may be the same as described throughout the disclosure and with respect to other methods mentioned herein. Terms user and patient are used interchangeably throughout the disclosure as the user of the mobile application is also a patient presented with their specific dental diagnostic data.

There may be implemented multiple ways of arranging the first area and the second area in the user interface. The first area may be above the second area in the user interface. In another example the first area may be below the second area in the user interface. In yet another example, the first area and the second area may be arranged mutually next to each other in the user interface. The first area and the second area may be arranged not to overlap in the user interface. Generally, the first area and the second area are used to denote different parts of the user interface. These areas may or may not be explicitly distinguished, for example within a specific graphical element of the user interface.

The method may further comprise detecting, in the second area, a region of the user's oral situation that corresponds to a part of the first digital 3D dental model affected by the selected dental condition, and highlighting the region of the user's oral situation visualized in the second area. In this way, the affected part of the user's oral situation is clearly distinguished, within the camera stream, from the rest of the user's oral situation.

Highlighting the region may comprise applying a color overlay and/or a bounding box to the region in the user's dental situation visualized in the second area.

Method of this embodiment may comprise recognizing objects in the video stream that correspond to objects of the first digital 3D dental model. Objects that may be recognized may be individual teeth, gingiva, tooth landmarks such as edges, cusps, grooves and/or dental conditions.

The first and/or the second digital 3D dental model may be segmented by the diagnostic module prior to being received by the mobile application. This means that the objects such as teeth, gingiva and dental conditions are already identified when the mobile application performs the disclosed methods.

Detecting of the region in the second area of the user interface may comprise applying a neural network trained to automatically label frames in a camera stream into dental conditions and/or other objects such as teeth or gingiva. The neural network may in this case be a Region Based Convolutional Neural Network (R-CNN) capable of labelling frames of a video stream. This neural network may be trained on training data comprising manually labeled images of dental objects, for example by expert dentists. The training data may comprise, in an example, more than a thousand labeled images. The manual labels may serve as ground truth data to which output of the neural network is compared to. The training process may be an iterative process in which a loss function is minimized to minimize a difference between the output of the neural network and the ground truth. During the training process, weights of the neural network may be adjusted until a value of the loss function satisfies a stopping criterion. One example of the loss function may be a mean-squared-error function. The minimization of the loss function may be achieved for example by using a stochastic gradient descent algorithm. The stopping criterion may be a specific threshold value for the loss function. The stopping criterion may be a number of epochs (training cycles) after which performance metrics cease to increase.

The first digital 3D dental model and the second digital 3D dental model may be stored on a memory of a mobile device hosting the mobile application. Alternatively, the first digital 3D dental model and the second digital 3D dental model may be stored on a remote memory and may be accessed by the mobile application based on a user input.

A data processing apparatus is further disclosed comprising means for carrying out the method comprising:
- receiving, in the user interface, a selection of a dental condition of the at least two dental conditions, and displaying buttons representative of digital 3D dental models available for comparison, wherein the buttons comprise a first button associated with a first digital 3D dental model representative of a patient's oral situation at a first point in time and a second button associated with a second digital 3D dental model representative of a patient's oral situation at a second point in time,
- receiving, in the user interface, a selection of the first button, and in response, displaying the first digital 3D dental model in a first area of the user interface, such that the displayed first digital 3D dental model visualizes the selected dental condition corresponding to the first point in time,
- arranging, in the user interface, a camera button configured, when engaged, to activate a front camera of a mobile device hosting the mobile application,
- detecting engaging of the camera button by a user, and in response, activating the front camera,
- displaying, in a second area of the user interface, a camera stream from the front camera such that the user, when facing the front camera, may compare the user's oral situation to the first digital 3D dental model.

A computer program product is further disclosed, comprising instructions which, when the program is executed by a processor, cause the computer to carry out the method comprising:
- receiving, in the user interface, a selection of a dental condition of the at least two dental conditions, and displaying buttons representative of digital 3D dental models available for comparison, wherein the buttons comprise a first button associated with a first digital 3D dental model representative of a patient's oral situation at a first point in time and a second button associated with a second digital 3D dental model representative of a patient's oral situation at a second point in time,
- receiving, in the user interface, a selection of the first button, and in response, displaying the first digital 3D dental model in a first area of the user interface, such that the displayed first digital 3D dental model visualizes the selected dental condition corresponding to the first point in time,
- arranging, in the user interface, a camera button configured, when engaged, to activate a front camera of a mobile device hosting the mobile application,
- detecting engaging of the camera button by a user, and in response, activating the front camera,
- displaying, in a second area of the user interface, a camera stream from the front camera such that the user, when facing the front camera, may compare the user's oral situation to the first digital 3D dental model.

A computer-readable medium is further disclosed, comprising instructions which, when executed by a processor, cause the processor to carry out a method comprising:
- receiving, in the user interface, a selection of a dental condition of the at least two dental conditions, and displaying buttons representative of digital 3D dental models available for comparison, wherein the buttons comprise a first button associated with a first digital 3D dental model representative of a patient's oral situation at a first point in time and a second button associated with a second digital 3D dental model representative of a patient's oral situation at a second point in time,
- receiving, in the user interface, a selection of the first button, and in response, displaying the first digital 3D dental model in a first area of the user interface, such that the displayed first digital 3D dental model visualizes the selected dental condition corresponding to the first point in time,
- arranging, in the user interface, a camera button configured, when engaged, to activate a front camera of a mobile device hosting the mobile application,
- detecting engaging of the camera button by a user, and in response, activating the front camera,
- displaying, in a second area of the user interface, a camera stream from the front camera such that the user, when facing the front camera, may compare the user's oral situation to the first digital 3D dental model.

A user interface of a mobile application for comparing a digital 3D dental model of a patient's oral situation to a user's oral situation is further disclosed, the user interface comprising:
- a button representative of a dental condition;
- buttons representative of digital 3D dental models available for comparison, wherein the buttons representative of digital 3D dental models comprise a first button associated with a first digital 3D dental model representative of a patient's oral situation at a first point in time and a second button associated with a second digital 3D dental model representative of the patient's oral situation at a second point in time;
- a first area configured to display the first digital 3D dental model based on a user selection of the first button and further configured to visualize the dental condition of the displayed first digital 3D dental model based on the user selection of the button representative of the dental condition;
- a camera button configured, when engaged, to activate a front camera of a mobile device hosting the mobile application;
- a second area configured to display a camera stream from the front camera, when the user engages the camera button such that the user, when facing the front camera, may compare the user's dental situation to the first digital 3D dental model.

### Brief description of the figures

FIG. 1 illustrates a user interface of a mobile application for reporting status on dental health according to the disclosure;
FIG. 2 illustrates a method for generating an individual dental profile according to the disclosure;
FIG. 3 illustrates an example of an individual dental profile;
FIGS. 4A - 4E illustrate the user interface of the mobile application with displayed metadata based on the individual dental profile;
FIGS. 5A - 5G illustrate the user interface of the mobile application with displayed dental routines generated based the individual dental profile;
FIGS. 6A - 6C illustrate examples of the user interface of the mobile application in scan compare mode;
FIG. 7 illustrates a method for comparing digital 3D dental models in the user interface of the mobile application;
FIGS. 8A-8B illustrate the user interface of the mobile application in dental mirror mode;
FIG. 9 illustrates a method for comparing a digital 3D dental model to a user's oral situation;
FIG. 10 illustrates a system according to the disclosure, comprising the mobile application.

### Detailed description

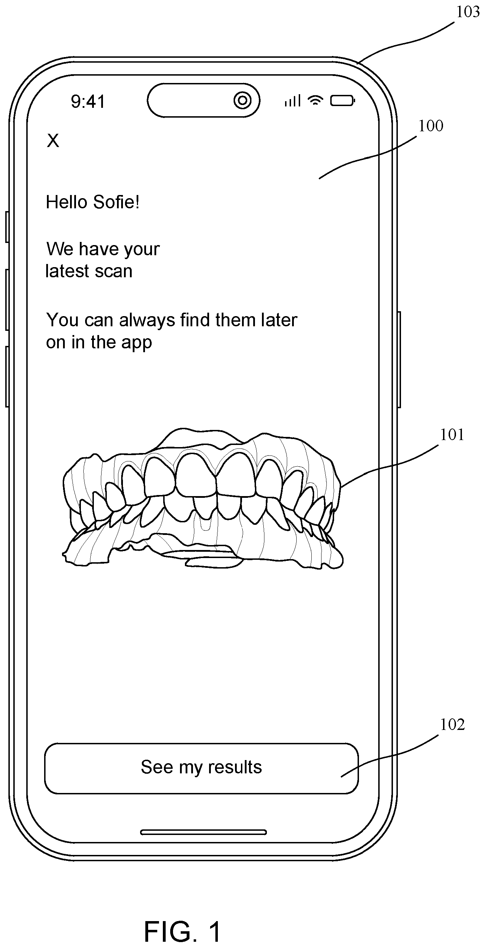

FIG. 1 illustrates a user interface 100 of a mobile application for reporting status on dental health of a patient. The mobile application may be hosted by a mobile device 103 shown as a mobile phone. Alternatively, the mobile device 103 may be a laptop or a tablet. Illustrated on FIG. 1 is the home page of the mobile application.

Displayed on the home page in the user interface 100 may be a digital 3D dental model 101 of the patient's oral situation. The digital 3D dental model 101 may comprise teeth and gingiva accurately representing those of the patient. The user of the mobile application (in this case the patient) may interact with the digital 3D dental model 101 by rotating and/or zooming in or out the model via a touchscreen display of the mobile device 103. In case there are multiple digital 3D dental models 101 of the patient available, then the most recent digital 3D dental model may be displayed automatically.

The user interface 100 may display a button 102 that, when engaged, may initiate displaying of the further details pertaining to the dental health of the patient.

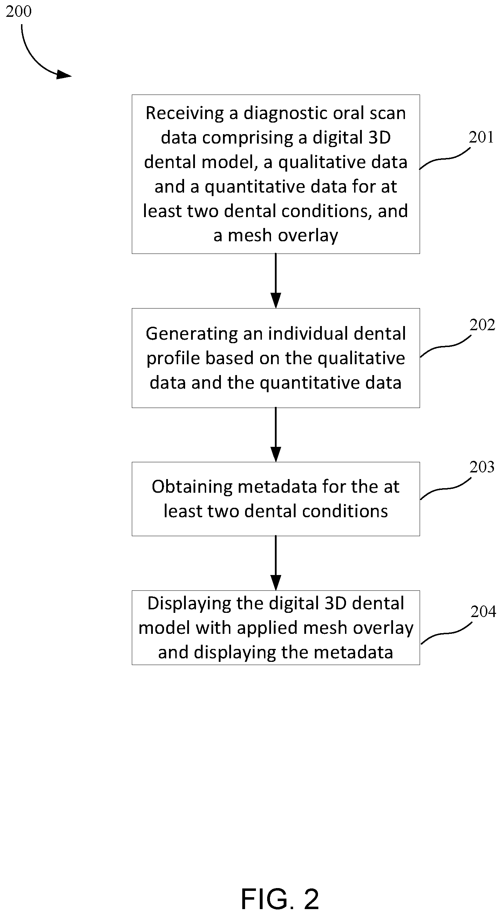

FIG. 2 illustrates a method 200 for generating an individual dental profile according to the disclosure. Step 201 illustrates receiving, by the mobile application, a diagnostic intraoral scan data of the patient's oral situation. The diagnostic intraoral scan data may comprise the digital 3D dental model 101 of the patient's oral situation, a quantitative data for at least two dental conditions associated with the patient's oral situation, a qualitative data for the at least two dental conditions, and a mesh overlay for each of the at least two dental conditions, wherein the mesh overlay is configured, when applied to the digital 3D dental model 101, to visualize the quantitative data and/or the qualitative data on the digital 3D dental model 101.

The diagnostic intraoral scan data may be stored at a remote location and may be received by the mobile application through a network such as the Internet. The diagnostic intraoral scan data may be obtained by applying one or more algorithms to intraoral scan data to identify presence and/or severity of dental conditions. These algorithms are not discussed in detail herein, but may include machine learning (ML) classification algorithms to classify building blocks of the digital 3D dental model 101, comprised in the intraoral scan data, into one of several dental condition categories.

The quantitative data for a dental condition may relate to a number of teeth affected by the dental condition, a number of lesions of the dental condition present, a percentage of total tooth surfaces affected, or a percentage of gingiva affected. The qualitative data may, on the other hand, relate to severity level of the lesions of a particular dental condition present in the oral situation. The qualitative data may be expressed per tooth of the oral situation or per individual lesion. The severity level may be one of internationally recognized severity levels for dental condition classification, such as International Caries Detection and Assessment System (ICDAS) classification for caries.

The mesh overlay may be a 3D color overlay that may be applied to the digital 3D dental model 101 to visually highlight parts of the digital 3D dental model 101 affected by a dental condition. Colors of the mesh overlay may indicate the qualitative data i.e. severity level of the dental condition. For example, red color may indicate severe level of the dental condition, orange may indicate moderate level while green may indicate initial severity level. Instead of colors, patterns or textures may be used to visualize lesions of the dental condition and/or their severity levels. The mesh overlay may be generated by a diagnostic module (discussed later) and may be an output of algorithms used to process the intraoral scan data to generate the diagnostic intraoral scan data.

Step 202 illustrates generating, by the mobile application, an individual dental profile 300 for the patient based on the quantitative data and/or the qualitative data. The individual dental profile 300 generated in this manner is reflective of the patient's dental health at a certain time. Generation of the individual dental profile 300 enables subsequent creation of a personalized care plan for the dental health of the patient.

Generating the individual dental profile 300 may comprise determining a representative severity value for each of the at least two dental conditions. Therefore, the representative severity value may be determined for at least two of caries, tooth wear, dental plaque, gingival recession, gingivitis, periodontitis, tooth cracks etc. In an example, the representative severity value for each of the at least two dental conditions may be computed as a weighted average of the quantitative data and/or the qualitative data for the each of the at least two dental conditions. The individual dental profile 300 may then comprise the representative severity values for each of the at least two dental conditions or may be determined as a function of the representative severity values.

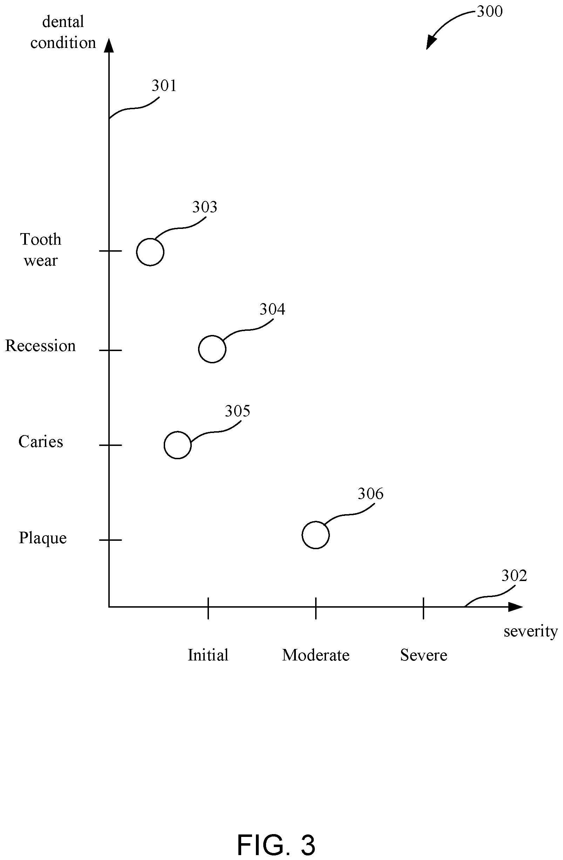

The individual dental profile 300 may be displayed in the user interface 100 of the mobile application in form of a dental score which may be a numerical value, in form of a 2D plot as shown in FIG. 3, in form of a chart such as a pie chart or in any other suitable way. The dental score may be determined as a function of the representative severity values of the at least two dental conditions, such as a weighted average. Instead of the weighted average, the dental score may be selected by a maximum of the representative severity values, taking the majority of the representative severity values or via a similar method. Converting the individual dental profile 300 into a single dental score may be advantageous as it can convey to the user the holistic status of the dental health, in form of a single value.

Step 203 of the method 200 illustrates obtaining, by the mobile application, metadata associated with the at least two dental conditions. The metadata may be curated based on the individual dental profile 300 for the purpose of providing relevant actionable recommendations and/or treatment plans to the patient. Therefore, the metadata may be tailored according to the specific individual dental profile 300. The metadata may be obtained from the official clinical sources such as World Health Organization (WHO), American Dental Association (ADA), National Health Service (NHS) or similar. The official clinical source may therefore be a local, regional, national or global health organization with expert knowledge in human dental health. The metadata may be curated by identifying correspondences of the metadata with the individual dental profile. The correspondences in this context may relate to correspondences between a representative severity value of a dental condition and metadata associated with such representative severity value of that dental condition. As an effect, only that information from the official clinical source which is relevant to the actual dental health of the patient is obtained. For example, if the representative severity value for the dental plaque is "moderate", then only information relevant to this particular condition and severity is sought after. Alternatively, the metadata may be curated based on the qualitative data and/or quantitative data.

Step 204 of the method 200 illustrates displaying, in the user interface 100 of the mobile application, the digital 3D dental model 101 with the applied mesh overlay, and further displaying the metadata. Additionally, the individual dental profile 300 may be displayed, either in a numerical form or as a diagram or chart.

FIG. 3 illustrates an example of the individual dental profile 300 in form of a two-dimensional plot. On a vertical axis 301 the at least two dental conditions may be indicated, in this case tooth wear, dental recession, caries and dental plaque are shown. On a horizontal axis 302 various severity levels associated with the at least two dental conditions may be shown. In the case of FIG. 3, three severity levels are shown: initial, moderate and severe, however different designations and number of severity levels may be used. The severity levels on the axis 302 may be representative severity values 303, 304, 305, 306 for each of the at least two dental conditions detected in patient's mouth. For example, the representative severity value for caries 305 describes the total severity of caries in the patient's oral situation and, as visible in FIG. 3, this value is lower than value "initial". The representative severity value may be determined individually for caries, tooth wear, dental plaque, gingival recession as in FIG. 3. In an example, the representative severity value 303, 304, 305, 306 for each of the at least two dental conditions may be computed as a weighted average of the quantitative data and/or the qualitative data for the each of the at least two dental conditions. The individual dental profile 300 may then comprise the representative severity values 303, 304, 305, 306 for each of the at least two dental conditions, or may be determined as a function of the representative severity values 303, 304, 305, 306.

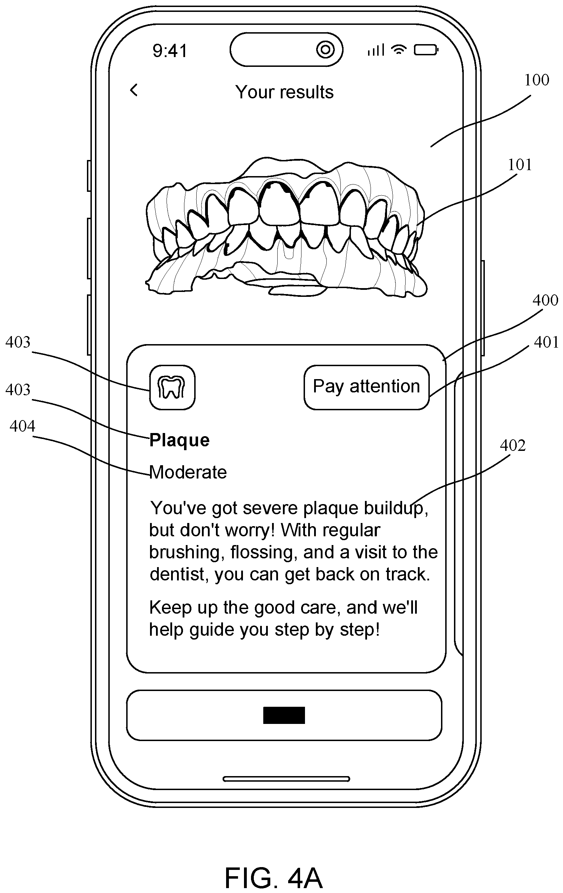

FIGS. 4A - 4E illustrate examples of the user interface 100 of the mobile application with displayed metadata 400, wherein the metadata 400 may be generated based on the individual dental profile 300. FIG. 4A shows the most severe dental condition which in this case is dental plaque of moderate severity. The displayed digital 3D dental model 101 comprises the applied mesh overlay which visualizes regions affected by the dental plaque. The metadata 400 may comprise a descriptor 401 which may be a general suggestion regarding the displayed dental condition. This general suggestion may be a function of the representative severity of the displayed dental condition. The metadata 400 may further comprise descriptive information 402 of the displayed dental condition. This descriptive information 400 may be generated for the user based on the individual dental profile 300. The metadata 400 may further comprise a dental condition identifier 403, in the text and/or symbol form, and the severity value 404 which may be the representative severity value 306 for plaque.

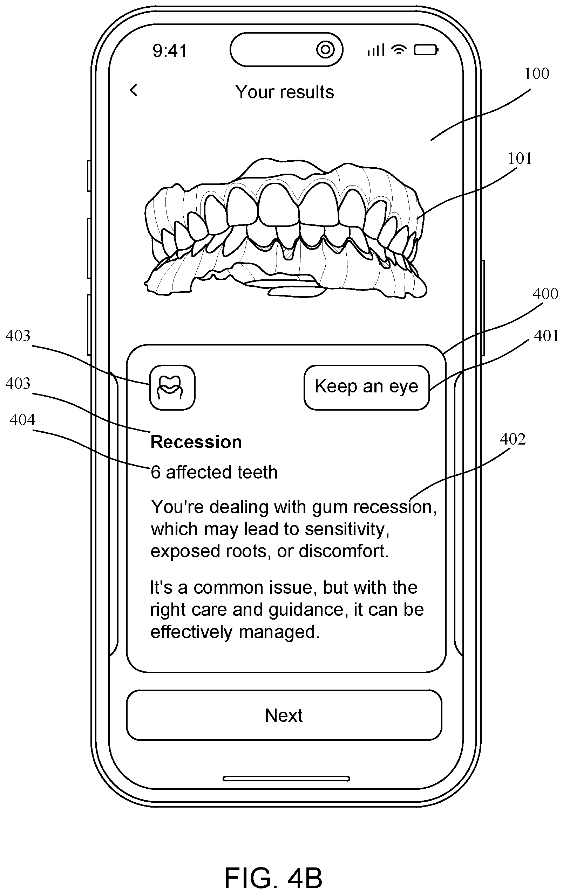

FIG. 4B illustrates the second most severe dental condition displayed in the user interface 100, which in this case is dental recession. The displayed digital 3D dental model 101 comprises the applied mesh overlay which visualizes regions affected by the dental recession. The metadata 400 may comprise the descriptor 401 which may be the general suggestion regarding the displayed dental condition. This general suggestion may be a function of the representative severity of the displayed dental condition. The metadata 400 may further comprise descriptive information 402 of the displayed dental condition. This descriptive information may be generated for the user based on the individual dental profile 300. The metadata 400 may further comprise a dental condition identifier 403, in the text and/or symbol form, and the severity value 404 which may be the number of teeth affected (quantitative data).

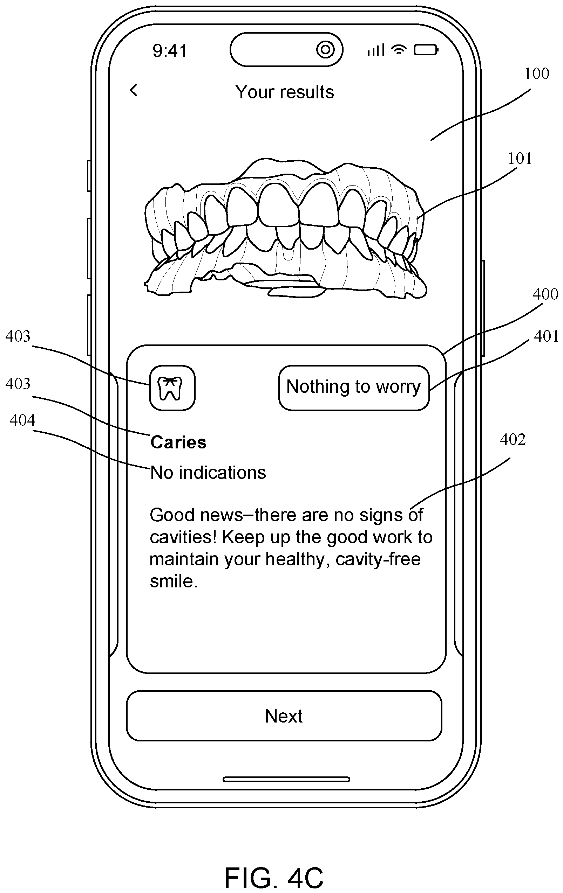

FIG. 4C illustrates a further dental condition displayed in the user interface 100 with a further lower severity level, which in this case is caries. The displayed digital 3D dental model 101 in this case does not comprise the applied mesh overlay as there were no identified caries lesions. The metadata 400 may comprise a corresponding descriptor 401 which may be a general suggestion regarding the displayed dental condition. This general suggestion may be a function of the representative severity of the displayed dental condition. The metadata 400 may further comprise descriptive information 402 of the displayed dental condition. This descriptive information may be generated for the user based on the individual dental profile 300. The metadata 400 may further comprise a dental condition identifier 403, in the text and/or symbol form, and the severity value 404 which in this case may have value "no indications" due to lack of identified caries lesions.

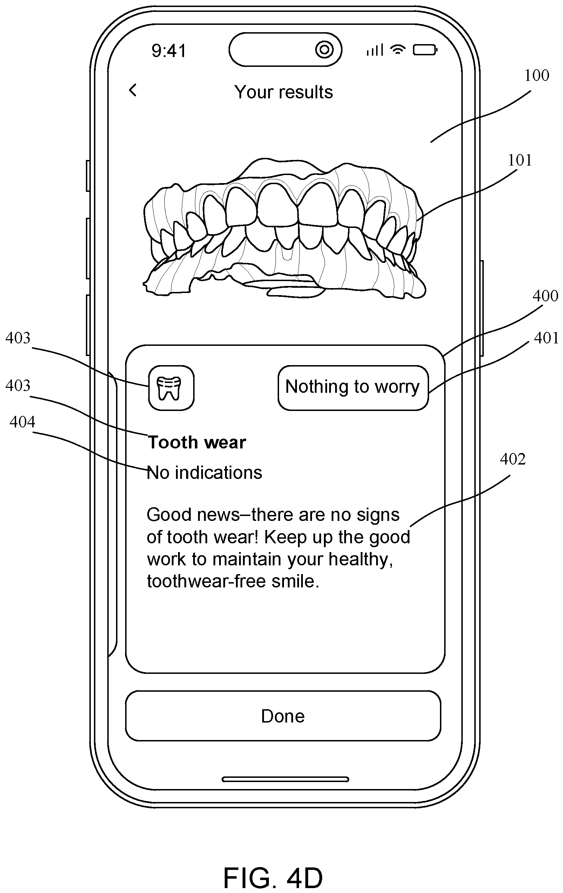

FIG. 4D illustrates a yet further dental condition with lower severity which in this case is tooth wear. The displayed digital 3D dental model 101 in this case does not comprise the applied mesh overlay as there was no identified tooth wear present. The metadata 400 may comprise the corresponding descriptor which may be a general suggestion regarding the displayed dental condition. This general suggestion may be a function of the representative severity of the displayed dental condition. The metadata 400 may further comprise descriptive information 402 of the displayed dental condition. This descriptive information may be generated for the user based on the individual dental profile 300. The metadata 400 may further comprise a dental condition identifier 403, in the text and/or symbol form, and the severity value 404 which in this case may have value "no indications" due to lack of identified tooth wear.

The metadata 400 on FIGS. 4A - 4D is shown in form of cards that can be navigated by the user of the mobile application with engaging the user interface 100 by a swipe motion (user can remove the displayed card by swiping the card) or through a designated button (e.g. button "Next"). In the illustrated example, only one card may be displayed at a time.

The descriptor 401 and/or the descriptive information 402 may be generated and presented so as to efficiently convey the most important information about the dental health to the patient.

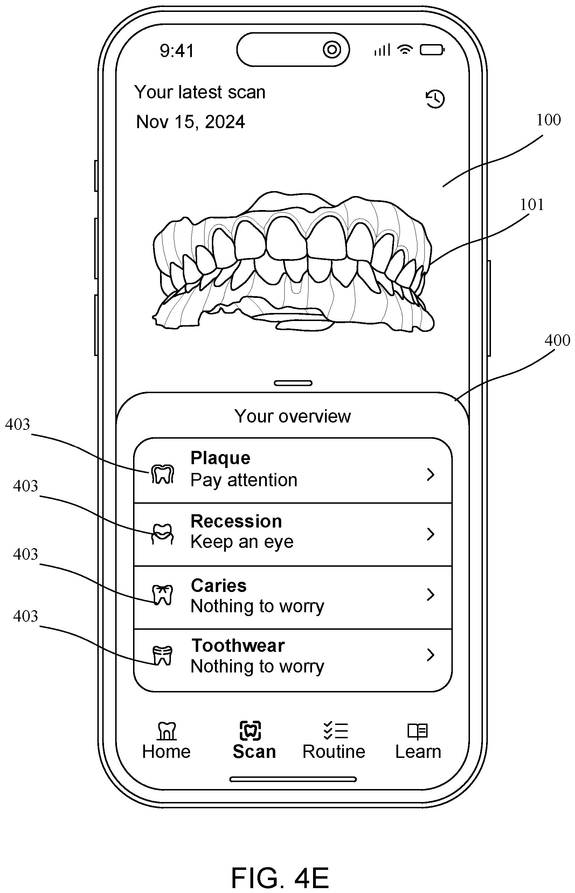

FIG. 4E illustrates an overview of the ranked dental conditions with their corresponding severity levels. The ranked dental conditions may be shown as part of the displayed metadata 400, ranked for example based on the corresponding representative severity. In the case of FIG. 4E, plaque is the most severe dental condition and is thus placed on the top of the overview in the metadata 400. Displayed additionally is the digital 3D dental model 101 representing the patient's oral situation which may automatically visualize the most severe dental condition, or may visualize a dental condition only after a corresponding user input (e.g. user selects a dental condition in the list of ranked dental conditions).

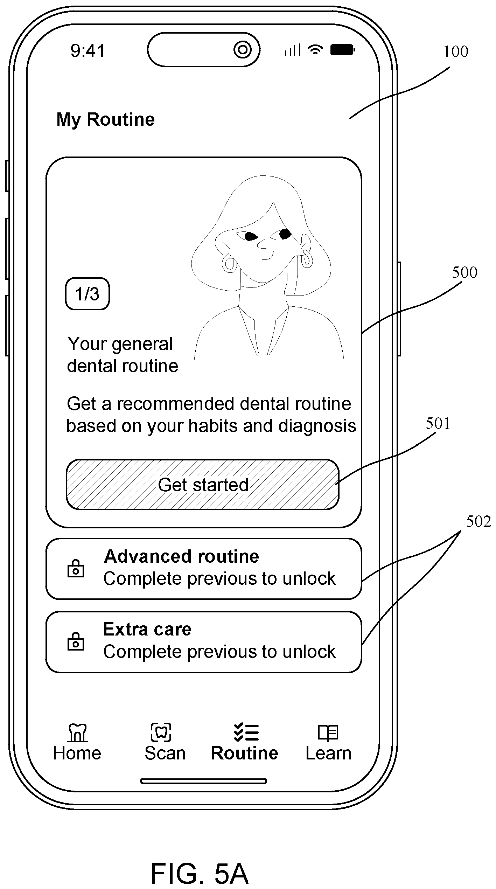

FIGS. 5A - 5G illustrate the user interface 100 of the mobile application during the process of registering user input regarding habits and subsequent generation of a recommended dental routine based on the user input. FIG. 5A illustrates a window 500, part of the user interface 100, which may be a starting point for generating the dental routine. Recording of the user input regarding the habits may be started by engaging a button 501 by the user. Buttons 502 may indicate advanced dental routine that can be generated for the user. Habits may relate to dental habits such as daily number of tooth brushing, flossing, use of mouthwash, type of toothbrush used etc.

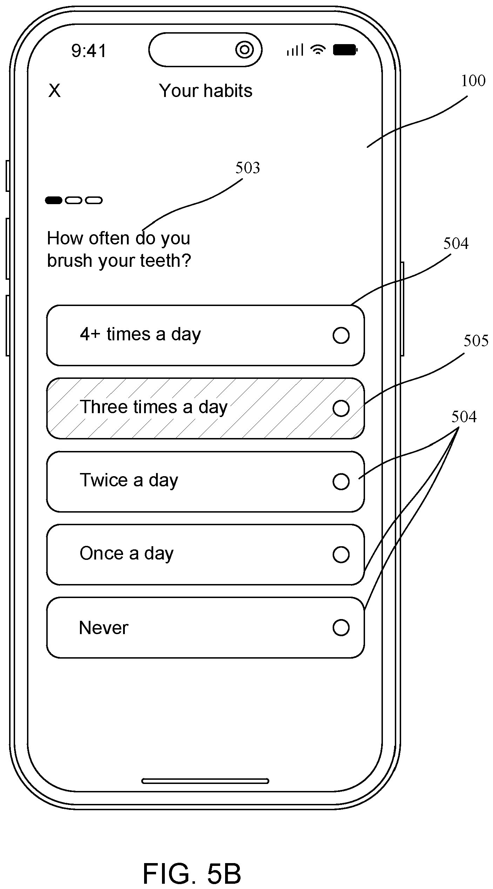

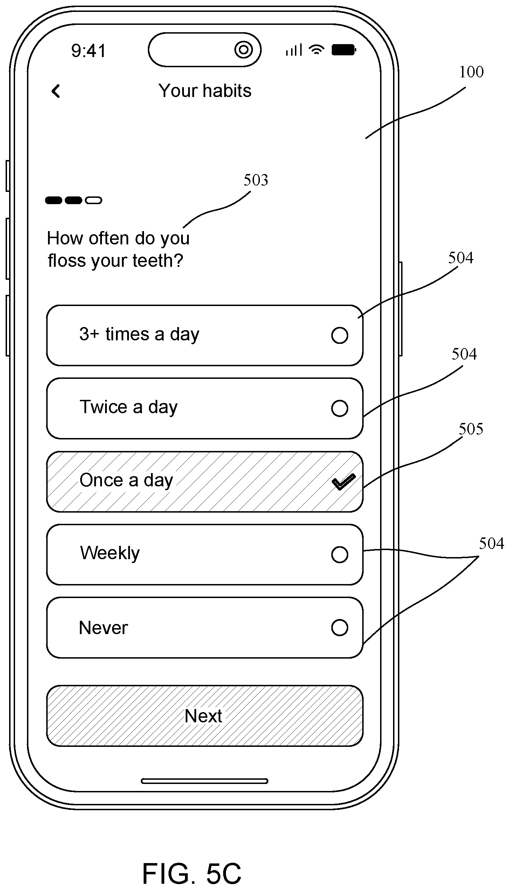

The user input regarding habits may be presented in form of a questionnaire comprising a question 503 and several provided answers 504, among which may be the user-selected answer 505, as shown in FIGS. 5B and 5C.

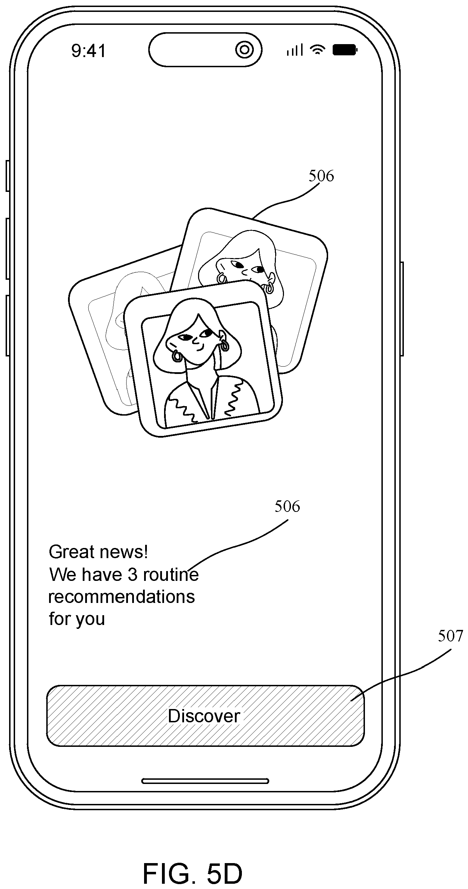

FIG. 5D visualizes a summary of recommended dental routines 506 indicating a number of dental routine recommendations generated based on the user input. The user, by engaging a button 507, may initiate displaying of the dental routines.

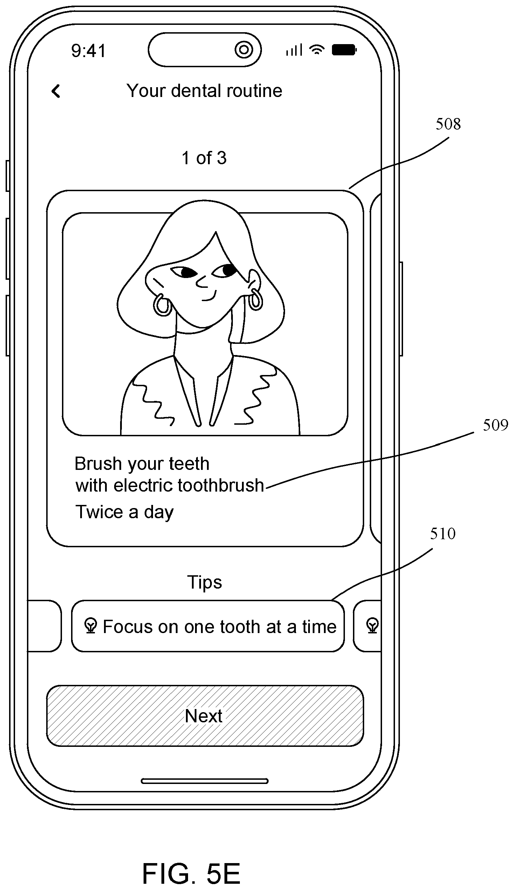

FIG. 5E illustrates a first dental routine 508 displayed in the user interface 100, that may comprise a recommendation 509 and a supplementary information 510 to the recommendation 509. The first dental routine 508 may be generated based on the individual dental profile 300 and/or the user input regarding habits. The first dental routine 508 may thus be correlated to the highest ranked dental condition and it may be selected such that it may have a highest relevance, or highest impact on the highest ranked dental condition. Relevance here should be understood as a potential of the recommended measure for reversal or containment of the dental condition.

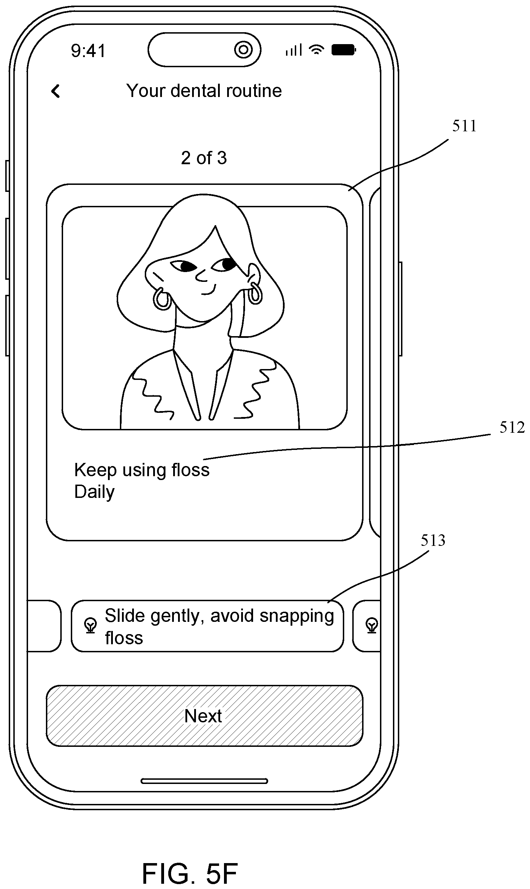

FIG. 5F illustrates a second dental routine 511 displayed in the user interface 100, that may comprise a second recommendation 512 and a second supplementary information 513 to the second recommendation 512. The second dental routine 508 may be of lesser importance to the impact on the highest ranked dental condition compared to the first dental routine 508.

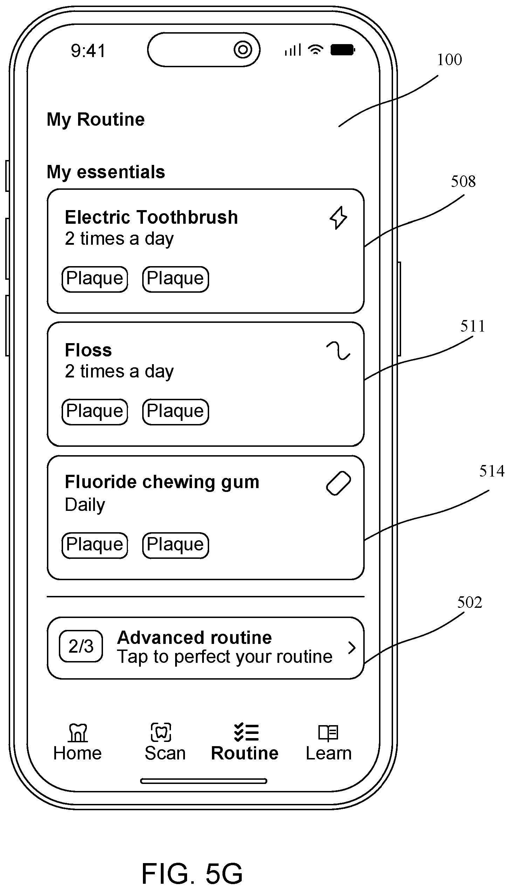

FIG. 5G illustrates the user interface 100 displaying simultaneously the first dental routine 508, the second dental routine 511 and a third dental routine 514, all of which may be generated based on the user input regarding the user's dental habits. The displayed routines 508, 511, 514 may be ranked based on their relevance to the highest ranked dental condition, in this case plaque, wherein the relevance may be seen as a potential of the recommended measure for reversal or containment of the dental condition. From the view of the mobile application presented in FIG. 5G, the user may engage the button 502 to activate advanced routine generation.

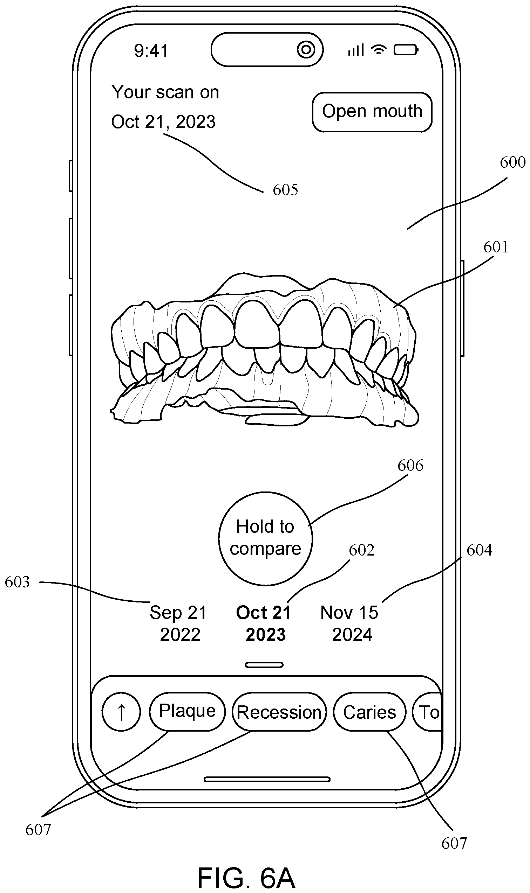

FIGS. 6A - 6C illustrate the user interface 100 of the mobile application in scan compare mode. Generally, the scan compare mode serves the user to efficiently compare the digital 3D dental models 101 from various time points in the user interface 100 of the mobile application. This gives the user a clear, visible overview of the changes in their oral situation over time. Several challenges occur when designing the user interface 100 of the mobile application for comparing digital 3D dental models. One of the challenges is to provide the user with an option to quickly select which digital 3D dental models should be compared among the available digital 3D dental models. It is generally desirable to design the user interface 100 such that it offers an effective way of selecting among the various available digital 3D dental models, as well as for the user interface 100 to display simultaneously the changes in the digital 3D dental models once the user makes the selection. A further challenge is to provide this content in a single user interface 100 on a limited surface area of the touchscreen display of the mobile device hosting the mobile application.

FIG. 6A illustrates a user interface 600 for scan comparison where a first digital 3D dental model 601 is displayed. This displayed digital 3D dental model 601 corresponds to the user's oral situation captured by an intraoral 3D scanner at a first time 605 which may be indicated in the user interface 600. The user interface 600 in this mode also comprises a first button 602 associated with the first digital 3D dental model 601. When the user selects the first button 602, the corresponding first digital 3D dental model 601 may be displayed. This selection may define one of the two digital 3D dental models for comparison.

Besides the first button 602, the user interface 600 for scan comparison may display a second button 603 associated with a second digital 3D dental model representative of the patient's oral situation at a second time, different from the first time 605 corresponding with the first digital 3D dental model 601. The user interface 600 may further comprise a third button 604 associated with a third digital 3D dental model representative of the patient's oral situation at a third time, different from the first time 605 and the second time. By selecting any of these buttons 602, 603, 604, the user interface changes 600 so that corresponding digital 3D model is displayed. By navigating back and forth through engaging individually the buttons 602, 603, 604, the user may observe interchange of displayed models, however detailed changes between the digital 3D models are difficult to observe in this manner. It is thus desirable to designate, via a single user gesture, a specific digital 3D model that will be compared with the first selected digital 3D dental model 601 and subsequently to highlight actual differences between these two digital 3D dental models. For this purpose, a compare button 606 may be arranged in the user interface 600.

The user may further engage a button 607 specifying a dental condition for which the differences between the two digital 3D dental models are to be displayed.

The user may designate either the second digital 3D dental model or the third digital 3D dental model to be compared with the first digital 3D dental model 601 in the following manner. The user may engage (or depress) the compare button 606 in the user interface 600 and subsequently perform a drag motion to the desired target for comparison, which may be the second button 603 or the third button 604. By pausing the drag motion at one of the second button 603 or the third button 604, the user selects the desired digital 3D dental model that will be compared to the first digital 3D dental model 601. This represents an effective way of enabling user input for selecting the desired digital 3D dental model that will be compared to the first digital 3D dental model 601. Described manner of user selection is particularly advantageous as the selection occurs within the same user interface 600 and with a single user gesture, thus allowing faster comparison of digital 3D dental models. The number of user interactions is therefore reduced, comparing to other methods of providing comparison of digital 3D dental models where a further selection menu needs to be opened. Further, the described method contributes to the user focus retention while simultaneously keeps the context of the information displayed in the user interface 600.

The arrangement of the compare button 606 and the buttons 602, 603, 604 is such that they are in vicinity of each other allowing the user to effectively perform the drag command. Furthermore, there are no other visual elements in between these buttons potentially obstructing the drag motion of the user.

Subsequently, the user interface 600 updates such that a transition from the initially displayed first digital 3D dental model 601 to the second (or third) digital 3D dental model is displayed. This transition may be a geometric morphing between the two digital 3D dental models or a transparency level adjustment. Alternatively, once the compare mode is activated a new digital 3D dental model may be displayed which may be a geometric superimposition of the two digital 3D dental models being compared, with the mutual geometric differences highlighted. In this manner, an efficient comparison method is provided allowing the user to preview, select the digital 3D dental models for comparison and observe the differences, all within the same user interface 600 and with a single user gesture. The described compare mode may be active in the period during which the user keeps the interaction with the desired target for comparison, i.e. while the user keeps the contact with either the second button 603 or the third button 604. Once the user releases the touchscreen, the user interface 600 may again render the first digital 3D dental model 601.

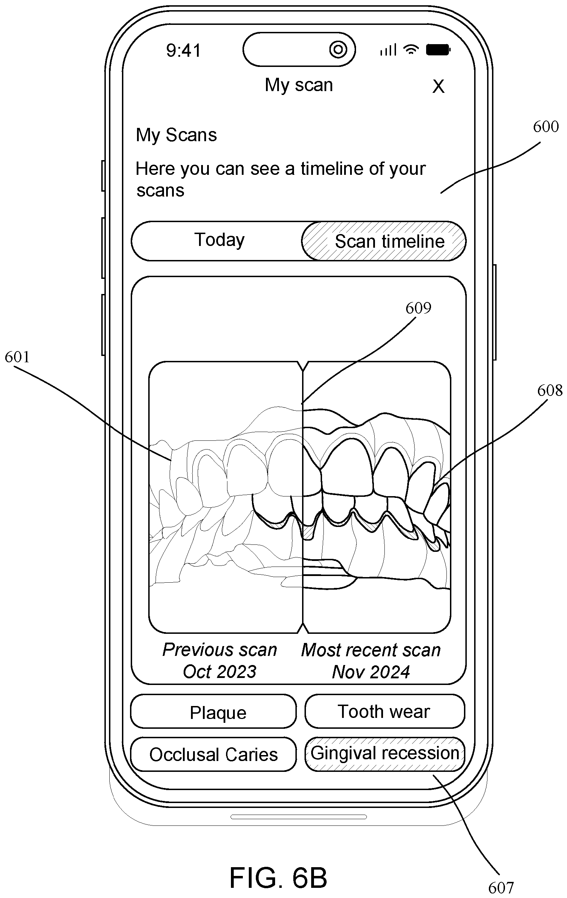

FIG. 6B illustrates a further example of the user interface 600 for scan comparison where the first digital 3D dental model 601 and the third digital 3D dental model 608 are displayed. The two digital 3D dental models 601, 608 may be displayed in a mutually superimposed manner and may be separated by a movable graphical user interface element 609, displayed in FIG. 6B as a straight line dividing the superimposed models. The user interface element 609 may be movable by the user, thus allowing the user to observe differences in a dental condition between the two digital 3D dental models 601, 608. The user may select the dental condition for which the differences between the two digital 3D dental models 601, 608 are to be displayed by engaging the button 607. In the case of FIG. 6B, the user has selected gingival recession to be highlighted on the superimposed digital 3D dental models 601, 608. An advantage of this manner of comparing digital 3D dental models is that local differences may be easier to observe, as the user has the ability to navigate the element 609 back and forth, thereby highlighting specific differences which otherwise may be difficult to observe.

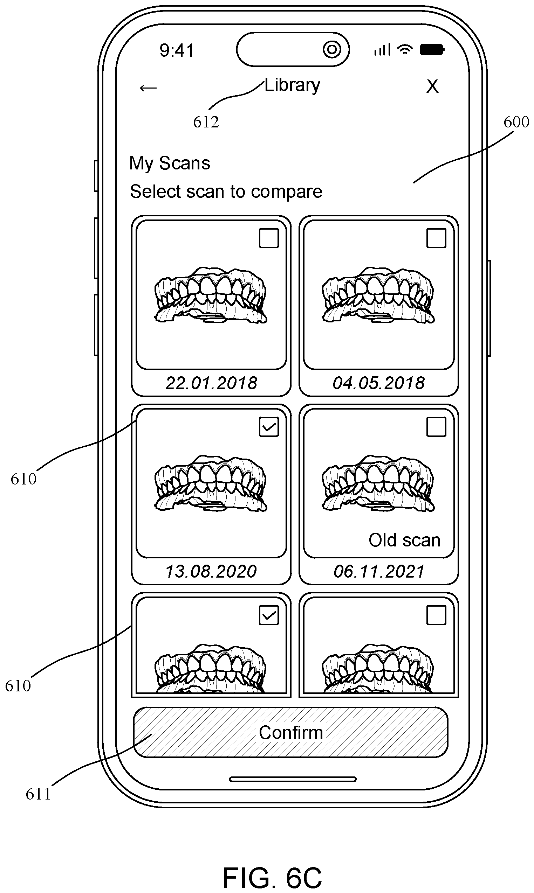

The user interface 600 for scan comparison may, in some examples, display a library 612 of available digital 3D dental models for mutual comparison, as seen in FIG. 6C. The user may, in the library 612, indicate two of the target digital 3D dental models 610 for which comparison is desired. By engaging a button 611 for confirming the user's choice, the user may activate comparing mode as illustrated for example on FIG. 6B. The library 612 enables efficient search and selection among available digital 3D dental models. A disadvantage however may be that the comparing process may be overall delayed by the fact that the user needs to navigate the library 612 and interact with multiple windows of the mobile application.

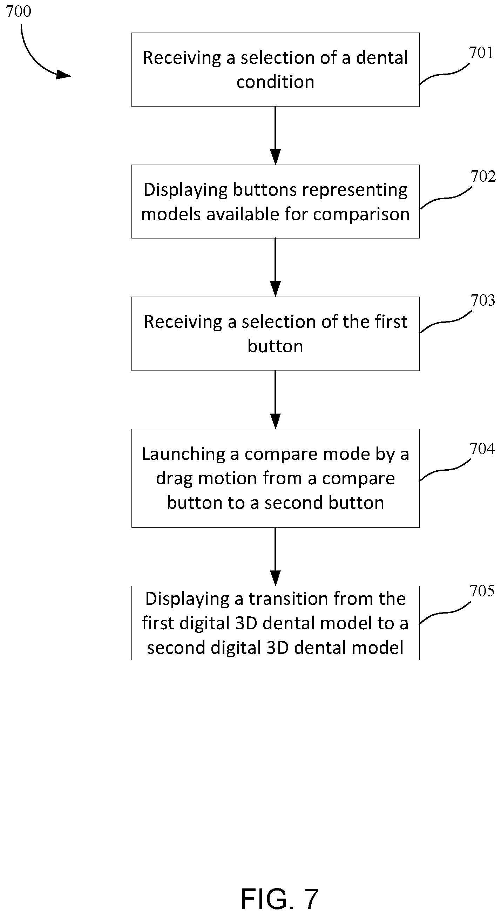

FIG. 7 illustrates a method 700 for comparing digital 3D dental models of the patient's oral situation according to an example.

Step 701 illustrates receiving, in the user interface 600 of the mobile application, a selection of the dental condition of the at least two dental conditions. In this way the user specifies the desired dental condition for which changes are to be tracked.

Step 702 illustrates displaying buttons 602, 603, 604 representative of digital 3D dental models available for comparison, wherein the buttons 602, 603, 604 comprise the first button 602 associated with the first digital 3D dental model 601 representative of the patient's oral situation at the first point in time and the second button 603 associated with the second digital 3D dental model representative of the patient's oral situation at the second point in time. The second time may be different than the first time.

Step 703 illustrates receiving, in the user interface 600, a selection of the first button 602, and in response, displaying the first digital 3D dental model 601 in the user interface 600, such that the displayed first digital 3D dental model 601 visualizes the selected dental condition corresponding to the first point in time. By executing this command, the user may select the first of the two digital 3D dental models for comparison.

Step 704 illustrates launching the compare mode of the mobile application by detecting the drag command performed by the user of the mobile application, the drag command comprising the drag motion, in the user interface 600, from the compare button 606 to the second button 603 (or to the third button). The user interface 600 with this particular arrangements of buttons and visual elements effectively guides the user to select the target digital 3D dental model for comparison. By executing this command, the user may select the second of the two digital 3D dental models for comparison.

Step 705 illustrates that in response to the drag command, displaying of the transition from the first digital 3D dental model 601 to the second digital 3D dental model may be performed, wherein the second digital 3D dental model visualizes the selected dental condition corresponding to the second point in time.

This described embodiment describes use of the drag command on the touchscreen of the mobile device hosting the mobile application for the purpose of comparing digital 3D dental models. Comparison of the user's various digital 3D dental models from different times is relevant for the user to visualize the progress of dental conditions over time. This disclosed method utilizes limited surface of the touchscreen economically, and provides for time-efficient way of comparing the digital 3D dental models and highlighting differences between those digital 3D dental models. This described method further solves the technical problem of simply identifying the digital 3D dental models for comparison and observing the differences between those digital 3D dental models all within the same user interface 600.

The transition from the first digital 3D dental model 601 to the second digital 3D dental model may comprise morphing the first digital 3D dental model 601 into the second digital 3D dental model. Alternatively, the transition from the first digital 3D dental model 601 to the second digital 3D model may comprise adjusting a transparency level of the first digital 3D dental model 601 to lower visibility of the first digital 3D dental model 601 and adjusting a transparency level of the second digital 3D dental model to increase visibility of the second digital 3D dental model to simulate the change from the first digital 3D dental model 601 into the second digital 3D dental model.

The compare mode may be active in a time period during which the user keeps interaction with the touchscreen, more precisely with the button 603, 604 representing the target digital 3D dental model. Once the user disengages from the touchscreen, the drag command may be interrupted and the compare mode may be closed. Upon closing of the compare mode, the user interface 600 may display transitioning back to the first digital 3D dental model 601. The method may thus further comprise detecting cessation of the drag command, and in response displaying a transition from the second digital 3D dental model to the first digital 3D dental model 601.

The method 700 therefore credibly assists the user in performing the task of comparing digital 3D dental models on a mobile device with a limited surface area of the touchscreen.

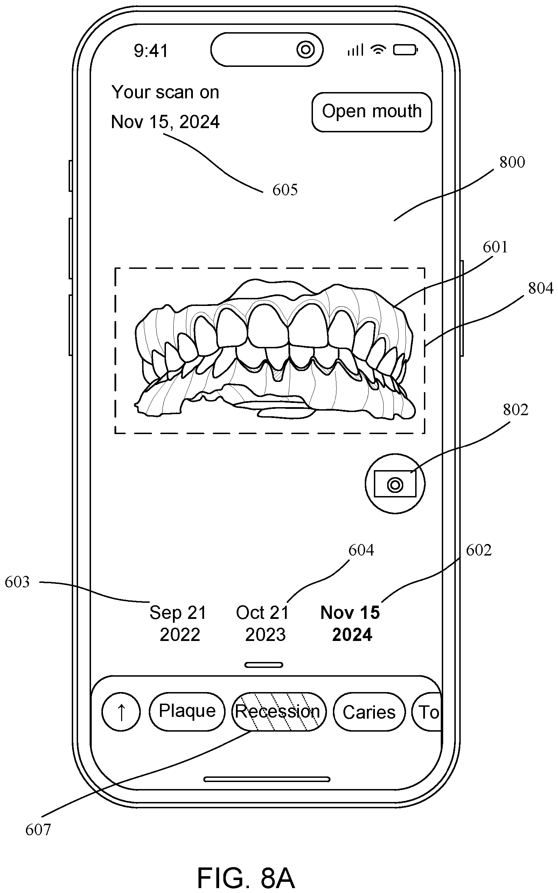

Once the user is presented with the status of a dental condition in the mobile application, such as in FIGS. 4A - 4F, the user may want to identify the diagnosed dental condition in their actual oral situation. FIGS. 8A - B illustrate the user interface 800 of the mobile application in a dental mirror mode. This mode of the mobile application allows the user to simultaneously see both the diagnosed dental condition on the corresponding displayed digital 3D dental model, and the actual oral situation, as will be presented herein. The user may, via the user interface and method presented here, advantageously use the mobile application as a digital dental mirror.

Shown in FIG. 8A is a user interface 800 in the digital dental mirror mode configured to simultaneously display a digital 3D dental model highlighting a dental condition, and the user's actual oral situation as captured by a camera of the mobile device hosting the mobile application.

Based on the user engaging the first button 602, the user interface 800 may display the first digital 3D dental model 601 corresponding to the user's oral situation captured by an intraoral 3D scanner at the first time 605 which may be indicated in the user interface 800. The user interface 800 in this mode also may comprise the second button 603 associated with the second digital 3D dental model representative of the patient's oral situation at the second time, different from the first time 605, and the third button 604 associated with a third digital 3D dental model representative of the patient's oral situation at the third time, different from the first time 605 and the second time. By selecting any of these buttons 602, 603, 604, the user interface 800 changes so that corresponding digital 3D model is displayed.

The first digital 3D dental model (or any other available model) may be displayed in a first area 804 of the user interface 800. The first area may comprise explicit borders or may have invisible borders.

The user may further engage a button 607 specifying the dental condition for displaying on the first digital 3D dental model 601 (in case of FIG. 8A recession is selected).

The user interface may further comprise a camera button 802 which, when engaged by the user, activates a camera stream from the front camera 801 such that the user, when facing the front camera 801, may compare the user's dental situation to the first digital 3D dental model 101 (or any other selected available digital 3D dental model). Once the front camera 801 is activated, a camera stream from the front camera 801 may be displayed in a second area 803 of the user interface 800. The second area 803 may be explicitly designated with borderlines or may have invisible borders. The second area 803 and the first area 804 may be mutually arranged not to overlap in the user interface 800. Equipped with these functionalities, the user interface 800 offers unique opportunity to the user to simultaneously track the actual oral situation and the corresponding digital 3D dental model 601.

The user may, in this manner, identify a region 805 in the user's oral situation that corresponds to a part 806 of the first digital 3D dental model 601 affected by the selected dental condition.

The user interface 800, and the mobile application overall, may be further improved by automatically detecting and highlighting the region 805 in the user's oral situation corresponding to the part 806 of the first digital 3D dental model 601 affected by the selected dental condition. The user is in this way automatically directed towards the relevant part of the oral situation suffering from the dental condition. A particular advantage of this feature is that conditions more difficult to observe and/or even those less severe cases of the dental condition, also difficult to observe by the user, may be explicitly and clearly pointed out to the user.

Automatic detection of the region 805 in the second area 803 of the user interface 800 may comprise applying a neural network trained to automatically label frames in the camera stream into dental conditions and/or other objects such as teeth or gingiva. The neural network may in this case be a Region Based Convolutional Neural Network (R-CNN) capable of labelling frames of a video stream. This neural network may be trained on training data comprising manually labeled images of dental objects, for example by expert dentists. The training data may comprise, in an example, more than a thousand, preferable more than five thousand labeled images. The manual labels may serve as ground truth data to which output of the neural network is compared to. The training process may be an iterative process in which a loss function is minimized to minimize a difference between the output of the neural network and the ground truth. During the training process, weights of the neural network may be adjusted until a value of the loss function satisfies a stopping criterion. One example of the loss function may be a mean-squared-error function. The minimization of the loss function may be achieved for example by using a stochastic gradient descent algorithm. The stopping criterion may be a specific threshold value for the loss function. The stopping criterion may, in an example, be a number of epochs (training cycles) after which performance metrics cease to increase.

Highlighting of the region 805 may be done in form of applying a bounding box around the region 805 or applying a color overlay onto the region 805.

In an example, instead of displaying the camera stream in the second area 803, the mobile application may be configured to automatically capture a 2D image of the user that corresponds to a view showing the first digital 3D dental model 601 in the first area 804. The 2D image may be automatically captured by the front camera 801 based on a correspondence criterion. The correspondence criterion may represent a relationship between the first digital 3D model 601 and the images of the video stream shown in the second area 803. When a correspondence between the first digital 3D model 601 and a 2D image from the video stream is detected, that 2D image may be automatically captured by the front camera 801. This correspondence detection may be based on object recognition within the video stream so that corresponding objects are identified with respect to objects of the displayed first digital 3D model 601.

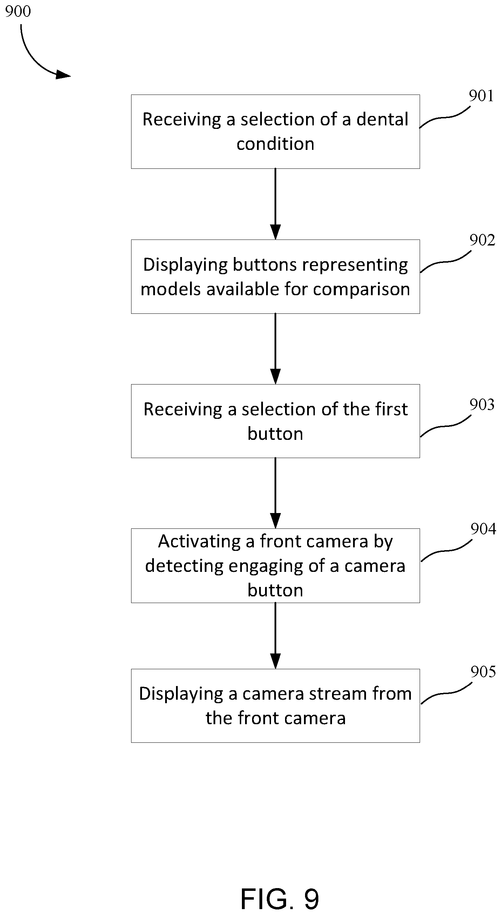

FIG. 9 illustrates a method 900 for comparing a digital 3D dental model to a user's oral situation.

The computer-implemented method 900 illustrates steps for comparing, in the user interface 800 of the mobile application, the digital 3D dental model of the patient's oral situation to the user's oral situation.

Step 901 illustrates receiving, in the user interface 800, a selection of the dental condition from the at least two dental conditions. By performing this action, the user specifies a target dental condition that they would like to observe within the user's oral situation.

Step 902 illustrates displaying buttons 602, 603, 604 representative of digital 3D dental models available for comparison, wherein the buttons 602, 603, 604 comprise the first button 602 associated with the first digital 3D dental model 601 representative of the patient's oral situation at the first point in time and the second button 603 associated with the second digital 3D dental model representative of the patient's oral situation at the second point in time.

Step 903 illustrates receiving, in the user interface 800, a selection of the first button 602, and in response, displaying the first digital 3D dental model 601 in a first area 804 of the user interface 800, such that the displayed first digital 3D dental model 601 visualizes the selected dental condition corresponding to the first point in time.

Step 904 illustrates activating the front camera 801 of the mobile device hosting the mobile application. The front camera 801 may be activated by engaging the camera button 802 arranged in the user interface 800.

Step 905 illustrates displaying, in the second area 803 of the user interface 800, the camera stream from the front camera 801 such that the user, when facing the front camera 801, may compare the user's dental situation to the first digital 3D dental model 101.

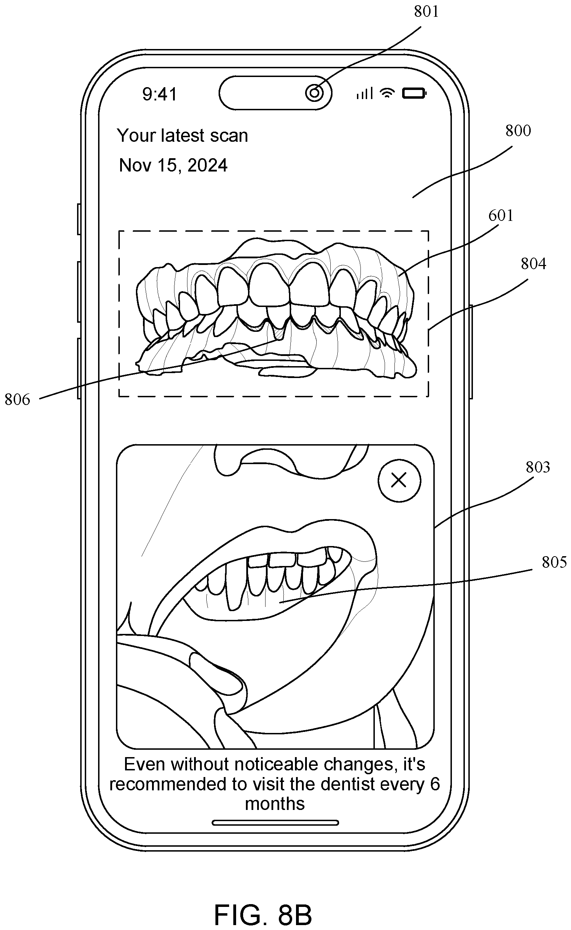

The user may in this way observe both their oral situation and the displayed digital 3D model 601visualizing the relevant dental condition, which in case of FIGS. 8A and 8B is dental recession. There may be multiple ways of arranging the first area 804 and the second area 803 in the user interface 800. The first area 804 may be above the second area 803 in the user interface 800, as shown in FIG. 8B. In another example the first area 804 may be below the second area 803 in the user interface 800. In yet another example, the first area 804 and the second area 803 may be arranged mutually next to each other in the user interface 800, for example horizontally next to each other. The first area 804 and the second area 803 may be arranged not to overlap in the user interface 800.

The method may further comprise detecting, in the second area 803, the region 805 in the user's oral situation that corresponds to a part 806 of the first digital 3D dental model 601 affected by the selected dental condition 607, and highlighting the region 805 in the user's oral situation visualized in the second area 803. This may comprise recognizing objects in the video stream that correspond to objects of the first digital 3D dental model 601. Highlighting the region may comprise applying the color overlay or the bounding box to the region in the user's dental situation visualized in the second area 803.

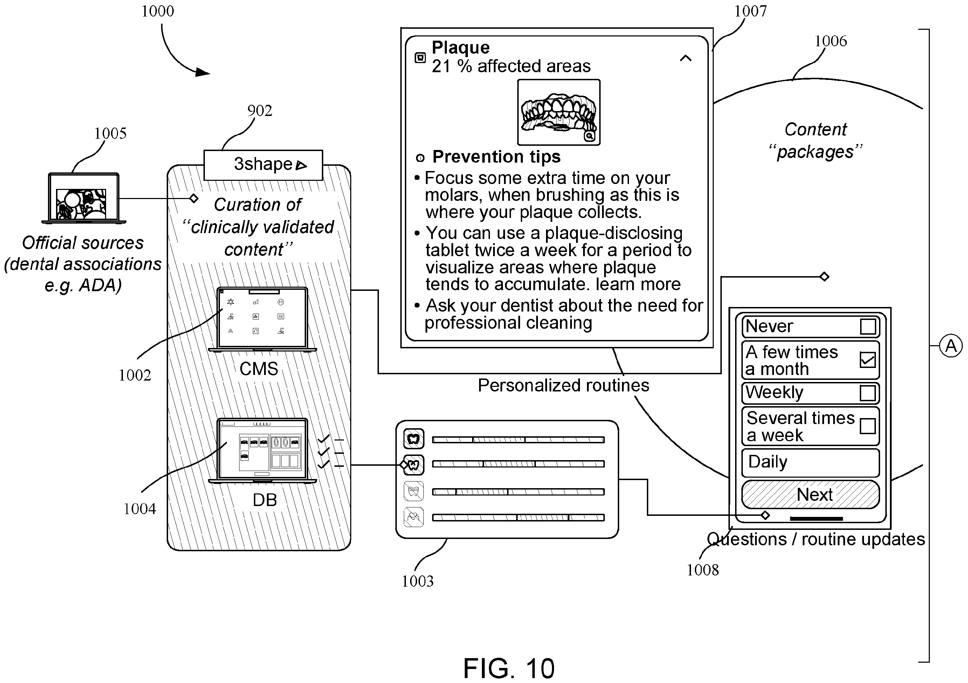

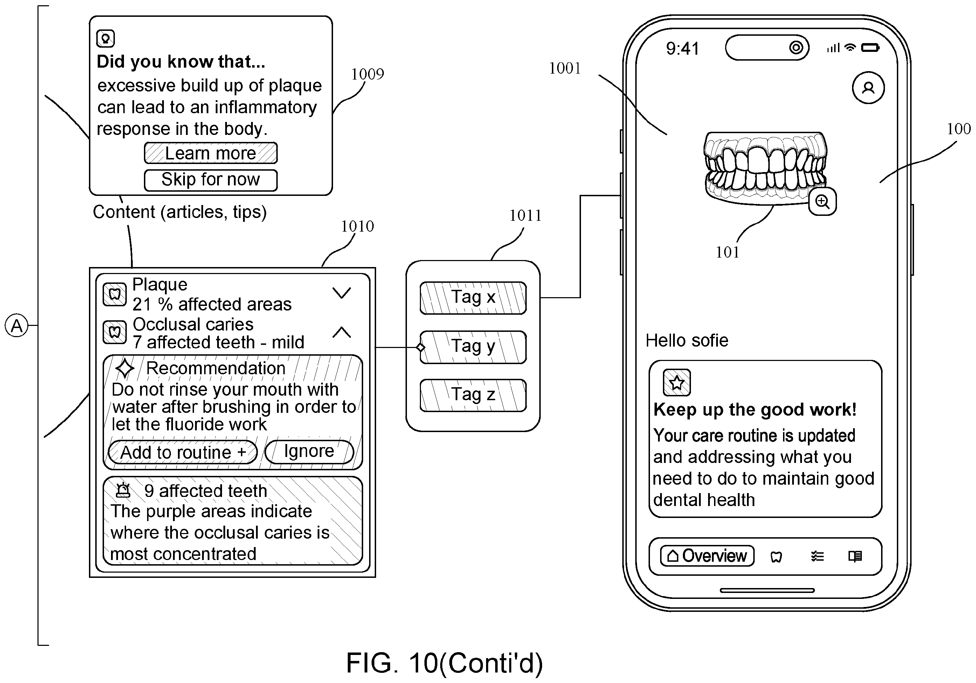

FIG. 10 illustrates a system 1000 according to the disclosure. The system 1000 may comprise the mobile application 1001 for communicating patient's diagnostic data and generating actionable recommendations for improving dental health. The system 1000 may further comprise the content management system 1002 which may be responsible for providing curated metadata associated with the at least two dental conditions of the patient. The content management system 1002 may be tasked with updating the content for the mobile application 1001 and thereby to increase user engagement. The system 1000 may further comprise the diagnostic module 1003 configured to generate the diagnostic intraoral scan data of the patient. The diagnostic module 1003 may thus be configured to receive intraoral scan data of the patient, including the digital 3D dental model 101, and to process the intraoral scan data so as to identify presence and/or severity values of the at least two dental conditions. The intraoral scan data may be stored in a database 1004 which may be a cloud database or a local server. The diagnostic module 1003 may further be configured to provide the diagnostic intraoral data and/or the digital 3D dental model 101 to the mobile application 1001. The diagnostic module 1003 may be configured to compress the digital 3D dental model before sending the digital 3D dental model to the mobile application 1001.

The mobile application 1001 may be configured to receive the diagnostic intraoral scan data of the patient comprising the digital 3D dental model 101 and to generate the individual dental profile 300 for the patient based on the received diagnostic intraoral scan data. The mobile application 1001 may further be configured to obtain, from the content management system 1002, the metadata associated with the at least two dental conditions, wherein the metadata may be curated based the individual dental profile 300.

The metadata may be obtained by the content management system 1002 from an official clinical source 1005 (for example WHO, ADA or other). The mobile application 1001 may further be configured to display, in the user interface 100, the digital 3D dental model 101 and the metadata.

The content management system 1002 and/or the diagnostic module 1003 may be configured to generate and/or update content packages 1006 for the mobile application 1001. A content package 1006 may comprise the metadata 1007, 1010 comprising diagnostic information on at least one dental condition, a questionnaire 1008 regarding the user's dental habits and/or symptoms as well as learning information 1009 which may comprise informative messages on dental conditions and further tips on dental health.

The content management system 1002 may be configured to provide metadata to the mobile application 1001 with a time delay. This may particularly be relevant when the content management system 1002 updates the provided metadata with new content. The time delay may be selected so to reflect the timing of starting and/or conducting a treatment regime that may be comprised in the metadata. Certain recommended actions within the treatment regime provided can only deliver maximum effectiveness if the user follows strict timeline of the treatment regime. In those cases it may be advantageous to set up providing of the metadata according to the recommended treatment regime. The time delay may alternatively or additionally be determined based on the individual dental profile 300.

Information provided to the mobile application 1001 may optionally pass through a sorting module 1011. The sorting module may, in an example, link the provided metadata 1007, 1010 to a corresponding dental condition in the mobile application 1001. The sorting module 1011 may perform such linking by applying a condition-specific tag on the metadata. FIG. 10 shows metadata 1007, 1010 associated with the dental plaque. The sorting module 1011 may link that metadata 1001, 1010 to the dental plaque in the mobile application 1001. The metadata already existing describing the dental plaque may be supplemented by the new metadata 1001, 1010 or may be partially or fully replaced. Alternatively or additionally, the sorting module 1011 may link the learning information 1009 comprised in a content package 1006 to the corresponding dental condition in the mobile application 1001.

The content management system 1002 may be configured to instruct the mobile application 1001 to request the user's input on dental habits and/or dental symptoms, for example by requesting input by means of a questionnaire 1008. The content management system 1002 may further be configured to curate the metadata before providing the metadata to the mobile application 1001. Curating the metadata may be performed by accessing the official clinical source 1005. Based on the individual dental profile 300 and/or the user's input on dental habits and/or dental symptoms, relevant information may be obtained from the official clinical source 1005 and adjusted by the content management system 1002, before being provided to the mobile application 1001.

The mobile application 1001 may be configured to compress the digital 3D dental model 101 before storing the digital 3D dental model 101 to a memory of the mobile device hosting the mobile application 1001.

The provided disclosure describes merely exemplary embodiments. As will be understood by those skilled in the art, the present disclosure may be embodied in various forms without departing from the spirit or essential characteristics thereof. The description is intended to be illustrative, but not limiting to the scope of the disclosure, as well as other claims. Throughout the disclosure, the word "comprising" does not exclude other elements or steps, and the indefinite article "a" or "an" does not exclude a plurality. The fact that certain features are recited in mutually different dependent claims does not indicate that a combination of these features cannot be used to advantage. It is to be understood that embodiments may be made, other than those mentioned, and structural and functional modifications may be made without departing from the scope of the present invention.

### List of items:

1. A computer-implemented method comprising:
   - receiving, by a mobile application, a diagnostic intraoral scan data of a patient's oral situation, wherein the diagnostic intraoral scan data comprises a digital 3D dental model of the patient's oral situation, a quantitative data for at least two dental conditions associated with the patient's oral situation, a qualitative data for the at least two dental conditions, and a mesh overlay for each of the at least two dental conditions, wherein the mesh overlay is configured, when applied to the digital 3D dental model, to visualize the quantitative data and the qualitative data on the digital 3D dental model;
   - generating, by the mobile application, an individual dental profile for the patient based on the quantitative data and the qualitative data;
   - obtaining, by the mobile application, metadata associated with the at least two dental conditions, wherein the metadata is curated based on the individual dental profile;
   - displaying, in a user interface of the mobile application, the digital 3D dental model with the applied mesh overlay, and further displaying the metadata.
2. The computer-implemented method of item 1, wherein the at least two dental conditions are at least two of: caries, tooth wear, dental plaque, gingival recession, gingivitis, periodontitis, tooth cracks.
3. The computer-implemented method according to any previous item, wherein the quantitative data comprises a number of teeth affected for each of the at least two dental conditions in the patient's oral situation.
4. The computer-implemented method according to the previous item 3, wherein the number of teeth affected is a percentage of total teeth surfaces if a dental condition of the at least two dental conditions is dental plaque.
5. The computer-implemented method according to any previous item, wherein the qualitative data comprises severity values for each of the at least two dental conditions in the patient's oral situation.
6. The computer-implemented method according to the previous item 5, wherein the severity values are defined for individual teeth and/or gingiva of the digital 3D dental model.
7. The computer-implemented method according to any previous item, wherein generating the individual dental profile comprises determining a representative severity value for each of the at least two dental conditions.
8. The computer-implemented method according to the previous item 7, wherein the representative severity value for each of the at least two dental conditions is computed as a weighted average of the quantitative data and the qualitative data for the each of the at least two dental conditions.
9. The computer-implemented method according to any previous item, further comprising displaying, in the user interface of the mobile application, the individual dental profile.
10. The computer-implemented method according to the previous item 9, wherein the individual dental profile is displayed in form of a dental score.
11. The computer-implemented method according to the previous item 10, wherein the dental score is determined as a function of the representative severity values of the at least two dental conditions.
12. The computer-implemented method according to the previous item 10 or 11, wherein the dental score is determined based on data collected from other users of the mobile application.
13. The computer-implemented method according to the previous item 12, wherein the data collected from other users of the mobile application comprises individual dental profiles of the other users.
14. The computer-implemented method according to any previous item, further comprising obtaining ranked dental conditions by ranking the at least two dental conditions based on the quantitative data and/or the qualitative data.
15. The computer-implemented method according to any previous item 7-14, further comprising obtaining ranked dental conditions by ranking the at least two dental conditions based on the representative severity values of the at least two dental conditions.
16. The computer-implemented method according to the previous item 14 or 15, further comprising displaying, in the user interface, the ranked dental conditions.
17. The computer-implemented method according to any previous item 14-16, wherein the displayed digital 3D dental model with the applied mesh overlay highlights a highest ranked dental condition of the ranked dental conditions.
18. The computer-implemented method according to the previous item 17, wherein the displayed digital 3D dental model with the applied mesh overlay indicates parts of the digital 3D dental model affected by the highest ranked dental condition.
19. The computer-implemented method according to any previous item 14-18, wherein the displayed metadata corresponds to the highest ranked dental condition of the ranked dental conditions.
20. The computer-implemented method according to any previous item 14-19, further comprising receiving a user selection of a dental condition other than the highest ranked dental condition and updating the applied mesh overlay such that the digital 3D dental model visualizes the selected dental condition.
21. The computer-implemented method according to the previous item 20, further comprising updating the displayed metadata such that the displayed metadata corresponds to the selected dental condition.
22. The computer-implemented method according to any previous item, wherein the metadata is obtained from an official clinical source.
23. The computer-implemented method according to the previous item 22, further comprising curating the metadata obtained from the official clinical source by identifying correspondences with the individual dental profile.
24. The computer-implemented method according to any previous item, wherein the metadata has been curated based on the qualitative data and/or quantitative data.
25. The computer-implemented method according to any previous item, further comprising annotating the diagnostic intraoral scan data with a demographic data of the patient.
26. The computer-implemented method according to any previous item, further comprising updating the individual dental profile with the demographic data of the patient.
27. The computer-implemented method according to the previous item 26, further comprising benchmarking the updated individual dental profile based on at least part of the demographic data.
28. The computer-implemented method according to any previous item 14-27, further comprising receiving, in the user interface, a user selection of a first dental condition of the at least two dental conditions and updating the user interface to display the metadata associated with the first dental condition.
29. The computer-implemented method according to the previous item 28, further receiving a user selection of a second dental condition of the at least two dental conditions, updating the displayed digital 3D dental model with a corresponding mesh overlay such that the digital 3D dental model visualizes the second dental condition and updating the user interface to display metadata associated with the second dental condition.
30. The computer-implemented method according to the previous item 29, wherein the user selection is recorded as a swipe motion on a display screen of a mobile device hosting the mobile application.
31. The computer-implemented method according to any previous item, further comprising receiving, by the mobile application, an external signal, and based on the received external signal deleting the quantitative and/or qualitative data for at least one dental condition of the at least two dental conditions.
32. The computer-implemented method according to any previous item, further comprising updating the user interface with a request for user's input on dental habits, receiving user's input on dental habits, and generating a recommended dental routine based on the received user's input on dental habits and/or the individual dental profile.
33. The computer-implemented method according to the previous item 32, wherein the recommended dental routine is displayed, in the user interface, for a dental condition of the at least two dental conditions.
34. The computer-implemented method according to the previous item 32, further comprising updating the individual dental profile based on the received user's input on dental habits.
35. The computer-implemented method according to any previous item 32-34, wherein generating the recommended dental routine further comprises accessing the official clinical source.
36. The computer-implemented method according to any previous item 32-35, wherein the recommended dental routine is displayed with a button, which when engaged, initiates a dialogue with a dentist.
37. The computer-implemented method according to any previous item, further comprising receiving a user input concerning dental symptoms and updating the individual dental profile based on the received user input concerning dental symptoms.
38. The computer-implemented method according to the previous item 37, further comprising updating the displayed metadata and/or the recommended dental routine based on the received user input concerning dental symptoms.
39. A data processing apparatus comprising means for carrying out the method according to any previous item 1-38.
40. A computer program product comprising instructions which, when the program is executed by a computer, cause the computer to carry out the method according to any previous item 1-38.
41. A computer-readable medium comprising instructions which, when executed by a computer, cause the computer to carry out the method according to any previous item 1-38.
42. A system comprising:
   - a mobile application;
   - a content management system;
   - a diagnostic module configured to generate a diagnostic intraoral scan data of a patient, wherein the mobile application is configured to:
   - receive, from the diagnostic module, the diagnostic intraoral scan data of the patient comprising a digital 3D dental model;
   - generate an individual dental profile for the patient based on the diagnostic intraoral scan data;
   - obtain, from the content management system, metadata based the individual dental profile; and
   - display, in a user interface of the mobile application, the digital 3D dental model and the metadata.
43. The system according to item 42, wherein the content management system is configured to provide metadata to the mobile application with a time delay.
44. The system according to the previous item 43, wherein the time delay is determined based on the individual dental profile.
45. The system according to the previous item 43, wherein the time delay is determined based on a recommended treatment regime.
46. The system according to any previous item 42-45, wherein the content management system is configured to instruct the mobile application to request the user's input on dental habits and/or dental symptoms.
47. The system according to any previous item 42-46, wherein the content management system is configured to curate the metadata before providing the metadata to the mobile application.
48. The system according to the previous item 47, wherein curating the metadata is performed by accessing an official clinical source.
49. The system according to any previous item 42-48, wherein the diagnostic module is configured to compress the digital 3D dental model before sending the digital 3D dental model to the mobile application.
50. The system according to any previous item 42-49, wherein the mobile application is configured to compress the digital 3D dental model before storing the digital 3D dental model to a memory of the mobile device hosting the mobile application.
51. The system according to any previous item 42-50 the content management system is configured to curate the metadata associated with the at least two dental conditions, based the individual dental profile.
52. A computer-implemented method for comparing digital 3D dental models of a patient's oral situation in a user interface of a mobile application, the method comprising:
   - receiving, in the user interface of the mobile application, a selection of a dental condition of the at least two dental conditions;
   - displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time;
   - receiving, in the user interface, a selection of the first button, and in response, displaying the first digital 3D dental model in the user interface, such that the displayed first digital 3D dental model visualizes the selected dental condition corresponding to the first point in time;
   - launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.
53. The computer-implemented method of item 52, wherein the transition from the first digital 3D dental model to the second digital 3D dental model comprises morphing the first digital 3D dental model into the second digital 3D dental model.
54. The computer-implemented method of item 52, wherein the transition from the first digital 3D dental model to the second digital 3D model comprises adjusting a transparency level of the first digital 3D dental model to lower visibility of the first digital 3D dental model and adjusting a transparency level of the second digital 3D dental model to increase visibility of the second digital 3D dental model.
55. The computer-implemented method of any previous item 52-54, wherein the transition from the first digital 3D dental model to the second digital 3D dental model highlights a difference between the first digital 3D dental model and the second digital 3D dental model, wherein the difference corresponds to a change in the selected dental condition.
56. The computer-implemented method of any previous item 52-55, further comprising detecting cessation of the drag command, and in response displaying a transition from the second digital 3D dental model to the first digital 3D dental model.
57. The computer-implemented method of any previous item 52-56, wherein the first digital 3D dental model is displayed with jaws closed.
58. The computer-implemented method of any previous item 52-57, further comprising receiving user input and based on the received user input displaying the first digital 3D dental model from an occlusal view.
59. The computer-implemented method according to the previous item 55, further comprising rotating the second digital 3D dental such that the difference is in a field of view of the user.
60. The computer-implemented method according to any previous item 52-59, wherein first digital 3D dental model and the second digital 3D dental model are stored on a memory of a mobile device hosting the mobile application.
61. The computer-implemented method according to any previous item 52-59, wherein first digital 3D dental model and the second digital 3D dental model are stored on a remote memory and are accessed by the mobile device hosting the mobile application based on a user input.
62. A user interface of a mobile application for tracking dental health of a user comprising:
   - buttons representative of digital 3D dental models available for comparison, wherein the buttons representative of digital 3D dental models comprise a first button associated with a first digital 3D dental model representative of a patient's oral situation at a first point in time and a second button associated with a second digital 3D dental model representative of the patient's oral situation at a second point in time, wherein the user interface is configured for displaying the first digital 3D dental model based on a user selection of the first button;
   - a compare button, wherein the user interface is configured to launch a compare mode of the mobile application by detecting a drag command performed by the user of the mobile application, the drag command comprising a drag motion, in the user interface, from the compare button to the second button, and in response to the drag command, display a transition from the first digital 3D dental model to the second digital 3D dental model.
63. The user interface of item 62, further comprising a button representative of a dental condition, wherein the user interface is configured to visualize the dental condition on the first digital 3D dental model or the second digital 3D dental model, based on the user selection of the button representative of the dental condition.
64. The user interface of item 63, wherein the dental condition is visualized as a surface color overlay.
65. The user interface of any previous item 62-64, wherein the transition from the first digital 3D dental model to the second digital 3D dental model comprises morphing the first digital 3D dental model into the second digital 3D dental model.
66. The user interface of any previous item 62-64, wherein the transition from the first digital 3D dental model to the second digital 3D model comprises adjusting a transparency level of the first digital 3D dental model to lower visibility of the first digital 3D dental model and adjusting a transparency level of the second digital 3D dental model to increase visibility of the second digital 3D dental model.
67. The user interface of any previous item 62-64, wherein the transition from the first digital 3D dental model to the second digital 3D dental model highlights a difference between the first digital 3D dental model and the second digital 3D dental model.
68. A data processing apparatus comprising means for carrying out the method according to any previous item 52-61.
69. A computer program product comprising instructions which, when the program is executed by a processor, cause the processor to carry out the method according to any previous item 52-61.
70. A computer-readable medium comprising instructions which, when executed by a processor, cause the processor to carry out the method according to any previous item 52-61.
71. A computer-implemented method for comparing, in a user interface of a mobile application, a digital 3D dental model of a patient's oral situation to a user's oral situation, the method comprising:
   - receiving, in the user interface, a selection of a dental condition of the at least two dental conditions;
   - displaying buttons representative of digital 3D dental models available for comparison, wherein the buttons comprise a first button associated with a first digital 3D dental model representative of a patient's oral situation at a first point in time and a second button associated with a second digital 3D dental model representative of a patient's oral situation at a second point in time;
   - receiving, in the user interface, a selection of the first button, and in response, displaying the first digital 3D dental model in a first area of the user interface, such that the displayed first digital 3D dental model visualizes the selected dental condition corresponding to the first point in time;
   - arranging, in the user interface, a camera button configured, when engaged, to activate a front camera of a mobile device hosting the mobile application;
   - detecting engaging of the camera button by a user, and in response, activating the front camera;
   - displaying, in a second area of the user interface, a camera stream from the front camera such that the user, when facing the front camera, may compare the user's dental situation to the first digital 3D dental model.
72. The computer-implemented method of item 71, wherein the first area is above the second area in the user interface.
73. The computer-implemented method of item 71, wherein the first area is below the second area in the user interface.
74. The computer-implemented method of item 71, wherein the first area and the second area don't overlap in the user interface.
75. The computer-implemented method of item 71, wherein the first area and the second area are arranged mutually next to each other in the user interface.
76. The computer-implemented method according to any previous item 71-75, further comprising detecting, in the second area, a region in the user's oral situation that corresponds to a part of the first digital 3D dental model affected by the selected dental condition, and highlighting the region in the user's oral situation visualized in the second area.
77. The computer-implemented method according to the previous item 76, wherein highlighting the region comprises applying a color overlay or a bounding box to the region in the user's dental situation visualized in the second area.
78. The computer-implemented method according to any previous item 76 or 77, wherein the detecting the region in the user's oral situation comprises applying a neural network trained to automatically label frames in a camera stream into dental conditions.
79. The computer-implemented method according to the previous item 78, wherein the neural network is a Region Based Convolutional Neural Network (R-CNN).
80. The computer-implemented method according to any previous item 71-79, wherein first digital 3D dental model and the second digital 3D dental model are stored on a memory of a mobile device hosting the mobile application.
81. The computer-implemented method according to any previous item 71-79, wherein first digital 3D dental model and the second digital 3D dental model are stored on a remote memory and are accessed by the mobile device hosting the mobile application based on a user input.
82. A data processing apparatus comprising means for carrying out the method according to any previous item 71-81.
83. A computer program product comprising instructions which, when the program is executed by a processor, cause the processor to carry out the method according to any previous item 71-81.
84. A computer-readable medium comprising instructions which, when executed by a processor, cause the processor to carry out the method according to any previous item 71-81.
85. A user interface of a mobile application for comparing a digital 3D dental model of a patient's oral situation to a user's oral situation comprising:
   - a button representative of a dental condition;
   - buttons representative of digital 3D dental models available for comparison, wherein the buttons representative of digital 3D dental models comprise a first button associated with a first digital 3D dental model representative of a patient's oral situation at a first point in time and a second button associated with a second digital 3D dental model representative of the patient's oral situation at a second point in time;
   - a first area configured to display the first digital 3D dental model based on a user selection of the first button and further configured to visualize the dental condition of the displayed first digital 3D dental model based on the user selection of the button representative of the dental condition;
   - a camera button configured, when engaged, to activate a front camera of a mobile device hosting the mobile application;
   - a second area configured to display a camera stream from the front camera, when the user engages the camera button such that the user, when facing the front camera, may compare the user's dental situation to the first digital 3D dental model.
86. The user interface of item 85, wherein the first area is arranged above the second area.
87. The user interface of item 85, wherein the first area is arranged below the second area.
88. The user interface of any previous item 85-87, wherein the first area and the second area don't overlap in the user interface.
89. The user interface of any previous item 85-88, wherein the first area and the second area are arranged mutually next to each other in the user interface.

## Claims

1. A computer-implemented method for comparing digital 3D dental models of a patient's oral situation in a user interface of a mobile application, the method comprising:
- receiving, in the user interface of the mobile application, a selection of a dental condition of the at least two dental conditions;
- displaying buttons representative of digital 3D dental models available for comparison, wherein the buttons comprise a first button associated with a first digital 3D dental model representative of a patient's oral situation at a first point in time and a second button associated with a second digital 3D dental model representative of the patient's oral situation at a second point in time;
- receiving, in the user interface, a selection of the first button, and in response, displaying the first digital 3D dental model in the user interface, such that the displayed first digital 3D dental model visualizes the selected dental condition corresponding to the first point in time;
- launching a compare mode of the mobile application by detecting a drag command performed by a user of the mobile application, the drag command comprising a drag motion, in the user interface, from a compare button to the second button, and in response to the drag command, displaying a transition from the first digital 3D dental model to the second digital 3D dental model, wherein the second digital 3D dental model visualizes the selected dental condition corresponding to the second point in time.

2. The computer-implemented method according to claim 1, wherein the transition from the first digital 3D dental model to the second digital 3D dental model comprises morphing the first digital 3D dental model into the second digital 3D dental model.

3. The computer-implemented method according to claim 1, wherein the transition from the first digital 3D dental model to the second digital 3D model comprises adjusting a transparency level of the first digital 3D dental model to lower visibility of the first digital 3D dental model and adjusting a transparency level of the second digital 3D dental model to increase visibility of the second digital 3D dental model.

4. The computer-implemented method according to any previous claim 1-3, wherein the transition from the first digital 3D dental model to the second digital 3D dental model highlights a difference between the first digital 3D dental model and the second digital 3D dental model, wherein the difference corresponds to a change in the selected dental condition.

5. The computer-implemented method according to any previous claim 1-4, further comprising detecting cessation of the drag command, and in response displaying a transition from the second digital 3D dental model to the first digital 3D dental model.

6. The computer-implemented method according to any previous claim 1-5, wherein the first digital 3D dental model is displayed with jaws closed.

7. The computer-implemented method according to any previous claim 1-6, further comprising receiving user input and based on the received user input displaying the first digital 3D dental model from an occlusal view.

8. The computer-implemented method according to any previous claim 4-7, further comprising rotating the second digital 3D dental such that the difference is in a field of view of the user.

9. The computer-implemented method according to any previous claim 1-8, wherein first digital 3D dental model and the second digital 3D dental model are stored on a memory of a mobile device hosting the mobile application.

10. The computer-implemented method according to any previous claim 1-8, wherein first digital 3D dental model and the second digital 3D dental model are stored on a remote memory and are accessed by the mobile device hosting the mobile application based on a user input.

11. A user interface of a mobile application for tracking dental health of a user comprising:
- buttons representative of digital 3D dental models available for comparison, wherein the buttons representative of digital 3D dental models comprise a first button associated with a first digital 3D dental model representative of a patient's oral situation at a first point in time and a second button associated with a second digital 3D dental model representative of the patient's oral situation at a second point in time, wherein the user interface is configured for displaying the first digital 3D dental model based on a user selection of the first button;
- a compare button, wherein the user interface is configured to launch a compare mode of the mobile application by detecting a drag command performed by the user of the mobile application, the drag command comprising a drag motion, in the user interface, from the compare button to the second button, and in response to the drag command, display a transition from the first digital 3D dental model to the second digital 3D dental model.

12. The user interface of claim 11, further comprising a button representative of a dental condition, wherein the user interface is configured to visualize the dental condition on the first digital 3D dental model or the second digital 3D dental model, based on the user selection of the button representative of the dental condition.

13. The user interface of claim 12, wherein the dental condition is visualized as a surface color overlay.

14. The user interface according to any previous claim 1-13, wherein the transition from the first digital 3D dental model to the second digital 3D dental model comprises morphing the first digital 3D dental model into the second digital 3D dental model.

15. The user interface of any previous claim 11-14, wherein the transition from the first digital 3D dental model to the second digital 3D model comprises adjusting a transparency level of the first digital 3D dental model to lower visibility of the first digital 3D dental model and adjusting a transparency level of the second digital 3D dental model to increase visibility of the second digital 3D dental model.
